# EUROPEAN PATENT SPECIFICATION

(11) **EP 2 566 967 B1**
(45) Date of publication and mention of the grant of the patent: **06.07.2016**
(21) Application number: 11778233.4
(22) Date of filing: 04.05.2011
(51) Int. Cl.: C12N 15/113, A61K 31/7088, A61K 39/00, A61K 38/17

(54) **CADHERIN-11 ANTAGONIST FOR THE TREATMENT OF FIBROSIS**
CADHERIN-11-ANTAGONISTEN ZUR BEHANDLUNG VON FIBROSE
ANTAGONISTE DE LA CADHÉRINE-11 POUR LE TRAITEMENT DE LA FIBROSE

(30) Priority: 04.05.2010 US 331355 P; 04.05.2010 US 331357 P
(43) Date of publication of application: 13.03.2013
(73) Proprietor: The Brigham and Women's Hospital, Inc., Boston, MA 02115 (US); Board of Regents, The University of Texas System, Austin, TX 78701 (US)
(72) Inventor: AGARWAL, Sandeep, K., Houston, TX 77005 (US); SCHNEIDER, Daniel, J., Ann Arbor Michigan (US); BRENNER, Michael, B., Newton, MA 02458 (US)
(74) Representative: Boult Wade Tennant
(86) International application number: PCT/US2011/035110
(87) International publication number: WO 2011/140173

(56) References cited:
- WO-A2-2009/101059
- WO-A2-2009/101059
- US-A1- 2009 253 200
- US-A1- 2010 093 602
- SELMAN MOISÉS ET AL: "Idiopathic pulmonary fibrosis: aberrant recapitulation of developmental programs?", PLOS MEDICINE, PUBLIC LIBRARY OF SCIENCE, US, vol. 5, no. 3, 4 March 2008 (2008-03-04), pages e62/1-8, XP009125316, ISSN: 1549-1676, DOI: 10.1371/JOURNAL.PMED.0050062
- GARDNER HUMPHREY ET AL: "Gene profiling of scleroderma skin reveals robust signatures of disease that are imperfectly reflected in the transcript profiles of explanted fibroblasts", ARTHRITIS & RHEUMATISM, JOHN WILEY & SONS, INC, US, vol. 54, no. 6, 1 June 2006 (2006-06-01), pages 1961-1973, XP002463683, ISSN: 0004-3591, DOI: 10.1002/ART.21894
- M. L. WHITFIELD ET AL: "Systemic and cell type-specific gene expression patterns in scleroderma skin", PROCEEDINGS OF THE NATIONAL ACADEMY OF SCIENCES, vol. 100, no. 21, 1 October 2003 (2003-10-01), pages 12319-12324, XP055076041, ISSN: 0027-8424, DOI: 10.1073/pnas.1635114100
- BINKLEY CHARLES E ET AL: "The molecular basis of pancreatic fibrosis - Common stromal gene expression in chronic pancreatitis and pancreatic adenocarcinoma", PANCREAS, RAVEN PRESS, NEW YORK, NY, US, vol. 29, no. 4, 1 November 2004 (2004-11-01), pages 254-263, XP009125254, ISSN: 0885-3177, DOI: 10.1097/00006676-200411000-00003
- D. J. SCHNEIDER ET AL: "Cadherin-11 contributes to pulmonary fibrosis: potential role in TGF- production and epithelial to mesenchymal transition", THE FASEB JOURNAL, vol. 26, no. 2, 1 February 2012 (2012-02-01), pages 503-512, XP055075080, ISSN: 0892-6638, DOI: 10.1096/fj.11-186098

## Description

### FIELD OF THE INVENTION

The invention provides a cadherin-11 antagonist for treating fibrosis by interfering with cadherin-11 activity, including cadherin-11 binding, as defined in the appended claims.

### BACKGROUND OF INVENTION

Interstitial lung diseases, now known as diffuse proliferative lung diseases, are a spectrum of diffuse parenchymal lung disease that is characterized by variable degrees of pulmonary fibrosis³. Pulmonary fibrosis is a component of various interstitial pneumonias³. These disorders are characterized by varying degrees of inflammation, aberrant fibroblast proliferation, and extracellular matrix deposition that result in distortion of pulmonary architecture that compromises pulmonary function^{3,4}. There are many causes of pulmonary fibrosis including exposure to fibrosis-inducing agents such as silica⁵, coal dust⁶, radiation and certain chemotherapeutic agents⁷. Despite its prevalence, the pathogenesis of pulmonary fibrosis is not completely understood and treatment options for the resolution of pulmonary fibrosis are lacking.

Approximately 35% of interstitial lung diseases can be grouped into a condition known as idiopathic pulmonary fibrosis (IPF)¹². A comprehensive epidemiological study investigating the incidence of IPF, revealed a general prevalence rate of approximately 20 cases per 100,000¹². This incidence was higher in individuals over 75 years of age where 175 cases per 100,000 were noted. A more recent study demonstrated a prevalence of approximately 43 cases per 100,000 and an incidence of 16.3 cases per 100,000 patient yearns.¹³ These data suggest that the incidence and prevalence of IPF in on the rise. IPF is a chronic and particularly devastating form of interstitial lung disease. It is largely untreatable and leads to death within 3 to 8 years of diagnosis¹⁴. There are no effective disease-modifying treatments for IPF.

WO2009/101059 describes that cadherin 11 is upregulated in endothelial-mesenchymal transition, in the early phase and late states of kidney fibrosis and in chronic allograft rejection kidneys.

### SUMMARY OF INVENTION

The invention is premised, in part, on the unexpected finding that cadherin-11 is involved in fibrotic conditions, including non-dermal fibrosis such as lung (or pulmonary) fibrosis, and that as a result cadherin-11 is a diagnostic marker, prognostic marker and therapeutic target for such conditions. As described in greater detail herein, cadherin-11 was found to be upregulated in fibrosis, and subjects lacking cadherin-11 were found to be less susceptible to experimentally-induced fibrosis. Accordingly, the disclosure provides compositions and methods for assessing the risk of developing fibrosis, as well as diagnosing, monitoring and treating fibrosis. The fibrosis may be, but is not limited to, non-dermal fibrosis including lung fibrosis.

The invention is further premised, in part, on another unexpected finding that cadherin-11 is expressed in alveolar macrophages and alveolar epithelial cells, both of which are present in bronchoalveolar lavage (BAL). Accordingly, it was found that the presence of lung fibrosis or the risk of developing lung fibrosis could be determined by analyzing cadherin-11 levels in BAL samples rather than requiring a biopsy of lung tissue. Such analysis may or may not comprise identifying the cells that express cadherin-11.

The invention provides a cadherin-11 antagonist:
(a) in an amount effective to reduce fibrosis for use in a method for treating a subject having fibrosis; or
(b) in an amount effective to prevent or delay the onset of symptoms associated with fibrosis for use in a method for treating a subject at risk of developing fibrosis;
wherein the cadherin-11 antagonist is a cadherin-11 binding peptide or a cadherin-11 nucleic acid antagonist.

In another embodiment, the fibrosis is non-dermal fibrosis. In one embodiment, the non-dernlal fibrosis is lung fibrosis or liver fibrosis. In some embodiments, the non-dermal fibrosis is lung fibrosis, more specifically idiopathic pulmonary fibrosis, and even more specifically severe idiopathic pulmonary fibrosis.

In one embodiment, the cadherin-11 antagonist is a cadherin-11 binding peptide. In one embodiment, the cadherin-11 binding peptide is an anti-cadherin-11 antibody or an antigen-binding antibody fragment. In one embodiment, the cadherin-11 binding peptide is a cadherin-11 fusion protein. In one embodiment, the cadherin-11 binding peptide comprises full length cadherin or a fragment thereof.

In one embodiment, the cadherin-11 antagonist is a cadherin-11 nucleic acid antagonist. In one embodiment, the cadherin-11 nucleic acid antagonist is a cadherin-11 siRNA. In one embodiment, the cadherin-11 nucleic acid antagonist is a cadherin-11 ribozyme. In one embodiment, the cadherin-11 nucleic acid antagonist is a cadherin-11 antisense molecule. In one embodiment, the cadherin-11 nucleic acid antagonist is a nucleic acid encoding full length cadherin-11 or a fragment thereof. In one embodiment, the cadherin-11 nucleic acid antagonist is an aptamer.

In one embodiment, the cadherin-11 antagonist is administered by inhalation or intranasally. In one embodiment, the cadherin-11 antagonist is administered intraperitoneally.

In one embodiment, the method further comprises administering to the subject a second therapeutic agent. In one embodiment, the second therapeutic agent is an immunosuppressant. In one embodiment, the immunosuppressant is a steroid.

In another aspect, the disclosure provides a method comprising measuring cadherin-11 level in a sample harvested from a subject, and comparing the cadherin-11 level in the sample with a normal control, wherein a cadherin-11 level in the sample harvested from the subject that is greater than the cadherin-11 level in the normal control indicates fibrosis or a risk of developing fibrosis. In one embodiment, the sample is a skin or dermis sample. In a related aspect, the disclosure provides a method comprising measuring cadherin-11 level in a sample harvested from a subject, and comparing the cadherin-11 level in the sample with a normal control, wherein a cadherin-11 level in the sample harvested from the subject that is greater than the cadherin-11 level in the normal control indicates non-dermal fibrosis or a risk of developing non-dermal fibrosis. In one embodiment, the sample is a lung sample. In one embodiment, the sample is a bronchoalveolar lavage (BAL) sample. In one embodiment, the sample comprises alveolar macrophages and/or alveolar epithelial cells. In one embodiment, the sample is a liver sample.

In various aspects, the cadherin-11 level is measured by manipulating the sample, including for example lysing cells within the sample and assaying the contents of such lysate using techniques known in the art.

In one embodiment, the cadherin-11 level is a cadherin-11 protein level. In one embodiment, the cadherin-11 level is a cadherin-11 mRNA level.

In one embodiment, the normal control is a sample of normal tissue or cells from the subject. In one embodiment, the normal control is a level of cadherin-11 in a population of subjects.

In one embodiment, cadherin-11 level is measured using immunohistochemistry.

In one embodiment, a cadherin-11 level in the sample harvested from the subject that is greater than the normal control indicates the subject has fibrosis. The fibrosis may be dermal fibrosis or non-dermal fibrosis such as lung fibrosis or liver fibrosis.

In one embodiment, a cadherin-11 level in the sample harvested from the subject that is greater than the normal control indicates the subject is at risk of developing fibrosis. The fibrosis may be dermal fibrosis or non-dermal fibrosis such as lung fibrosis or liver fibrosis.

In another aspect, the disclosure provides a method for inhibiting development (or differentiation) of myofibroblasts from fibroblasts using cadherin-11 antagonists. In important embodiments, fibroblast-to-myofibroblast differentiation is associated with development of fibrosis. The degree of inhibition may be determined by measuring the absolute or relative number or percentage of myofibroblasts following contact with the cadherin-11 antagonist. In some instances, inhibiting may include delaying (or slowing the kinetics of) myofibroblast development.

In another aspect, the disclosure provides a method for inhibiting an epithelial-to-mesenchymal transition (EMT). In important embodiments, the EMT is associated with development of fibrosis. In some instances, inhibiting may include delaying (or slowing the kinetics of) EMT. Markers of EMT include phenotype (e.g., loss of epithelial cell polarity, separation of cells from each other, expression of growth factors, (e.g., TGF-beta and wnt), expression of transcription factors (e.g., SNAILS, SMAD, LEF, and nuclear beta-catenin), expression of cell adhesion molecules (e.g., E-cadherin), and the like. These markers are known to those of ordinary skill in the art.

### BRIEF DESCRIPTION OF DRAWINGS

It is to be understood that the Figures are not necessarily to scale, emphasis instead being placed upon generally illustrating the various concepts discussed herein.

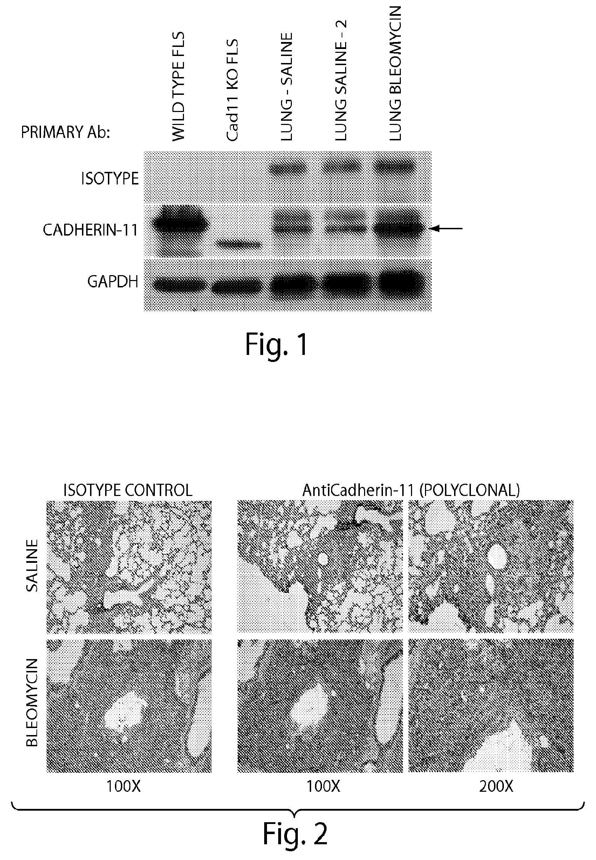

FIG. 1. Protein lysates were isolated from lungs of wild type mice administered saline or bleomycin via the intratracheal route. Lungs were harvested on day 12. Protein lysates were separated on a 10% acrylamide gel by electrophoresis, then transfer to a membrane for western blotting using isotype control antibody, anti-cadherin-11 antibody (Invitrogen) or anti-GAPDH antibody. Wild type and cadherin-11 null fibroblast-like synoviocytes were used as positive and negative controls for the expression of cadherin-11. The bleomycin lung had increased levels of cadherin-11 but similar levels of GAPDH, indicating that cadherin-11 is increased during the process of lung fibrosis.
FIG. 2. Immunohistochemical analyses of lungs of wild type mice administered saline or bleomycin via the intratracheal route. Lungs were harvested on day 12. Lung sections from mice administered intratracheal bleomycin demonstrated prominent staining of cadherin-11 on fibroblast-like cells in the areas that are forming fibrotic foci (red stain).

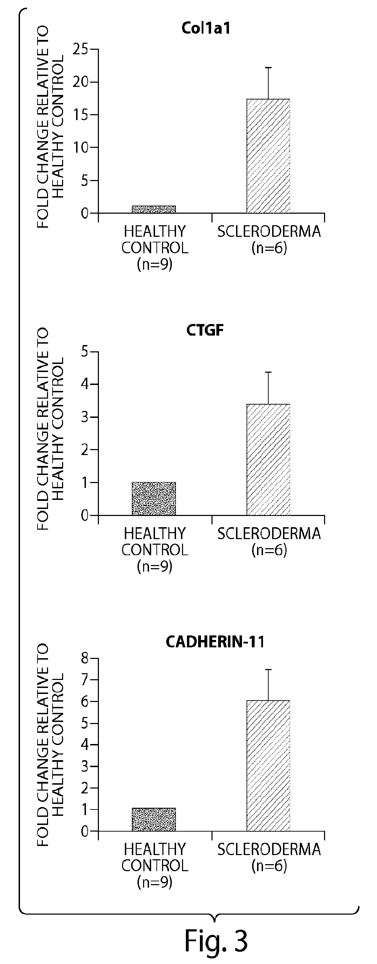

FIG. 3. Increased expression of cadherin-11 in skin biopsies from scleroderma patients (n=6) relative to healthy control subjects (n=9). Total RNA was isolated from skin biopsies and assessed for levels of col1a1 and CTGF, two genes associated with fibrosis, as well as Cadherin-11.

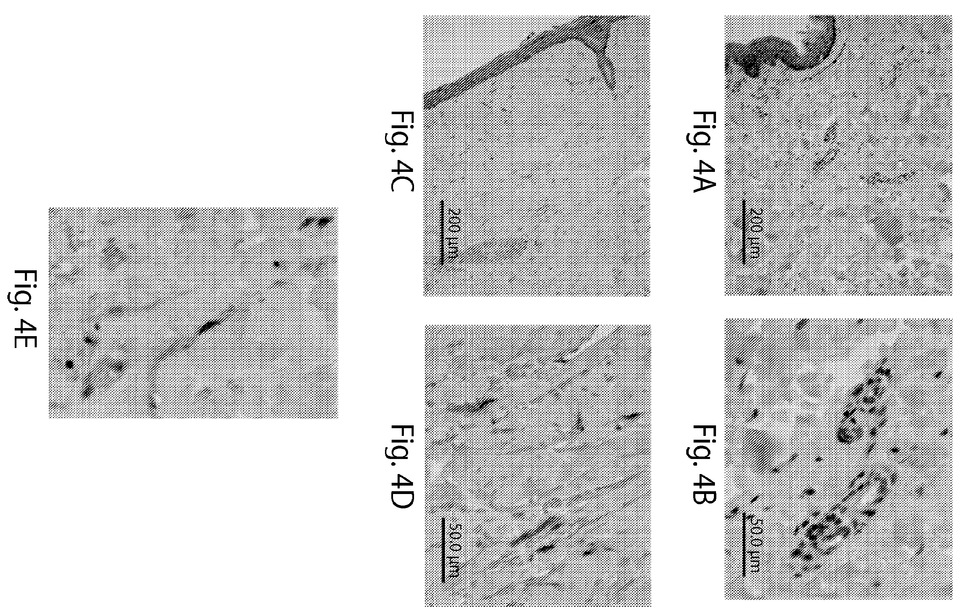

FIG. 4. Immunohistological staining of scleroderma and healthy control skin biopsies for cadherin-11. Skin biopsies stained with rabbit polyclonal anti-cadherin-11 antibody followed by HRP-conjugated secondary antibody. Positive cells are labeled with reddish brown color. (A) Normal skin low power magnification stained with anti-cadherin-11 antibody. (B) Normal skin high power magnification stained with anti-cadherin-11 antibody. (C) SSC skin low power magnification stained with anti-cadherin-11 antibody. (D) and (E) SSC skin high power magnification stained with anti-cadherin-11 antibody. Sections are representative of 4 control and 4 SSC skin biopsies.

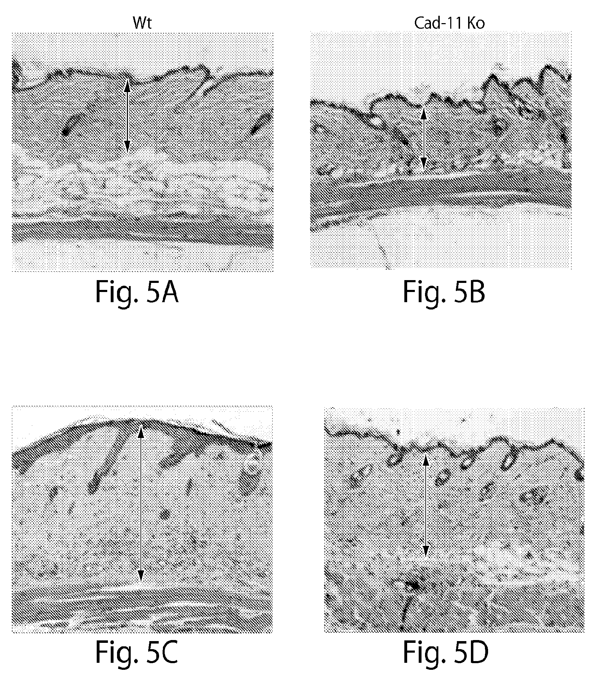

FIG. 5. Representative skin biopsies from mice in the bleomycin dermal fibrosis model stained with hematoxylin and eosin. (A) Wild type mice injected with PBS (n=7 mice), (B) Cad-11 KO mice injected with PBS (n=8 mice), (C) Wild type mice injected with bleomycin (n=11 mice), (D) Cad-11 KO mice injected with bleomycin (n=10 mice).

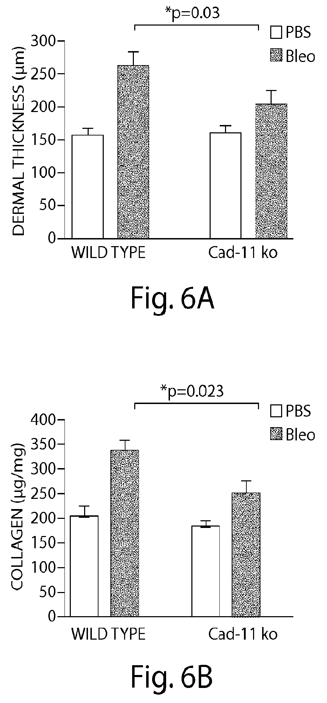

FIG. 6. Quantitation of dermal thickness (A) and fibrosis (B, collagen content as determined by Sircol Assay) in the bleomycin dermal fibrosis model. n=6 wild type mice injected with PBS, n=8 wild type mice injected with bleomycin, n=7 cad-11 KO mice injected with PBS, n=8 cad-11 KO mice injected with bleomycin (n=10 mice). Data are given as mean, error bars represent standard error of the mean. P-value determined using Student T-test comparing wild type and cad-11 KO mice injected with bleomycin.

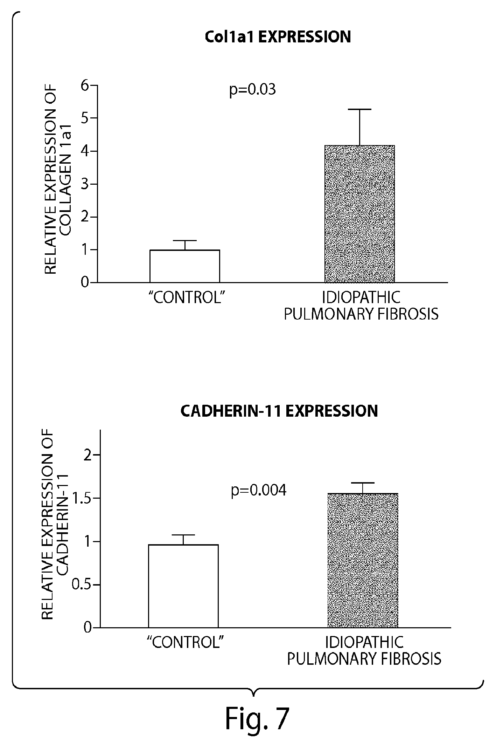

FIG. 7. mRNA levels of Col1a1 and cadherin-11 in lung tissue are increased in patients with severe IPF. Lung tissue was obtained from the Lung Tissue Research Consortium from 8 subjects with mild IPF and normal lung function (control, blue) and 10 subjects with severe IPF and abnormal lung function (IPF, red). Col1a1 was used as a control and was increased in patients with severe IPF. Cadherin-11 levels were also increased in patients with severe IPF. P-values determined using Student T-test.

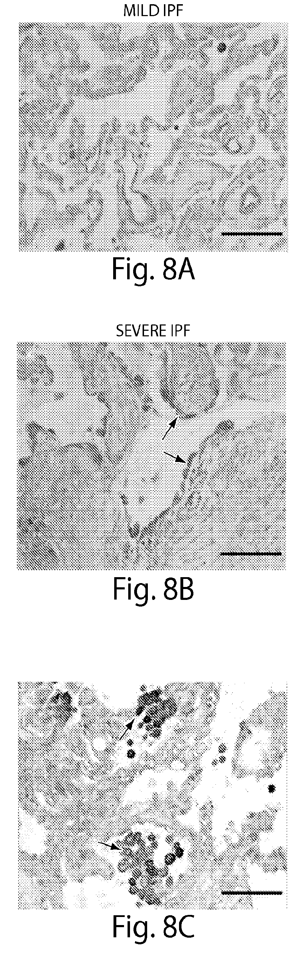

FIG. 8. Immunolocalization of cadherin-11 expression in the lungs of mild (A) and severe IPF patients (B,C). Arrows denote staining present in alveolar macrophages (C) and hyperplastic alveolar epithelial cells (B). Scale bars = 100 µm. Displayed sections are representative of n = 10 (mild IPF) and n = 10 (severe IPF).

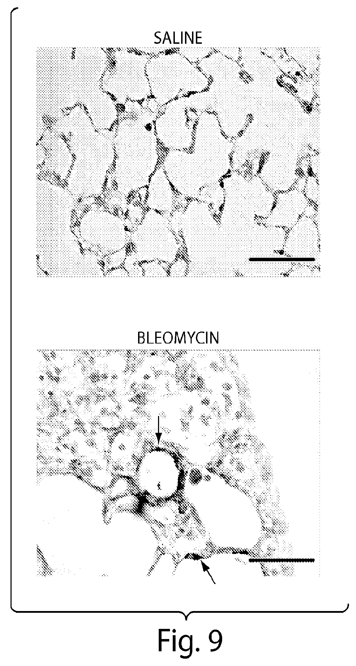

FIG. 9. Immunolocalization of cadherin-11 expression in the lungs of wild type mice administered intratracheal saline or bleomycin Arrows denote staining present in hyperplastic alveolar epithelial cells. Scale bars = 50 µm. Displayed sections are representative of n = 12 (saline) and n = 20 (bleomycin).

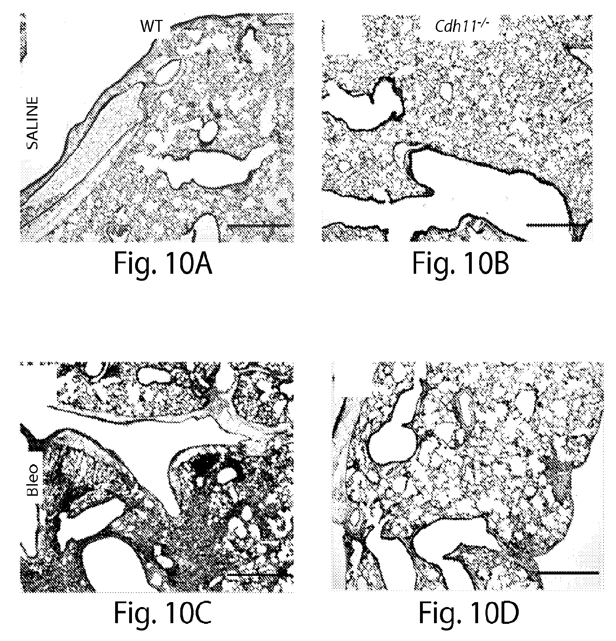

FIG. 10. Cadherin-11-dependent histopathology in pulmonary fibrosis. Lung histological sections from wild type and Cdh11 ^{-/-} mice in the bleomycin-induced pulmonary fibrosis model. Lungs were taken from mice 21 days after 3.5U bleomycin installation and processed for sectioning and H&E staining. (A) wild type mice administered intratracheal saline (n=6), (B) Cdh11 ^{-/-} mice administered intratracheal saline (n=6), (C) Wild type mice administered intratracheal bleomycin (n=13), (D) Cdh11 ^{-/-} mice administered intratracheal saline (n=11). Scale bars = 500 µm. Sections are representative of n = 6 WT saline, n = 6 Cdh11 ^{-/-} saline, n = 13 WT bleomycin, n = 11 Cdh11 ^{-/-} bleomycin.

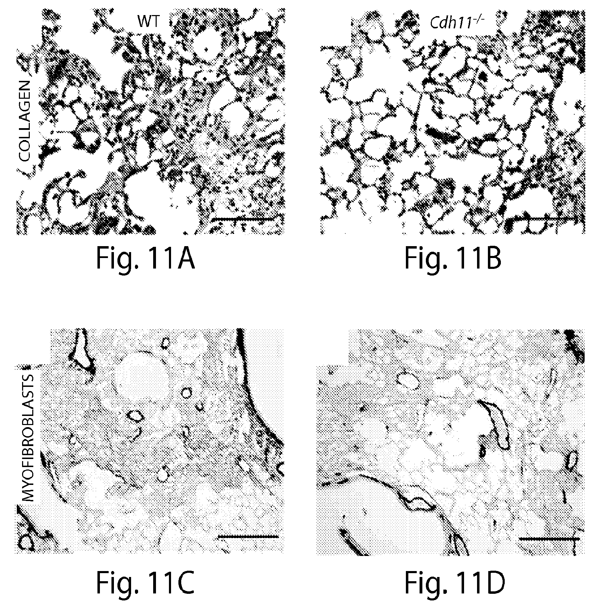

FIG. 11. Cadherin-11-dependent histopathology in pulmonary fibrosis. Lung histological sections from wild type and Cdh11 ^{-/-} mice in the bleomycin-induced pulmonary fibrosis model. Lungs were taken from mice 21 days after 3.5U bleomycin installation and processed for sectioning and Masson's trichrome stain (A,B) or IHC analyses for alpha smooth muscle actin, a marker for myofibroblasts (C,D). Scale bars = 200 µm. Sections are representative of n = 13 WT bleomycin, n = 11 Cdh11 ^{-/-} bleomycin.

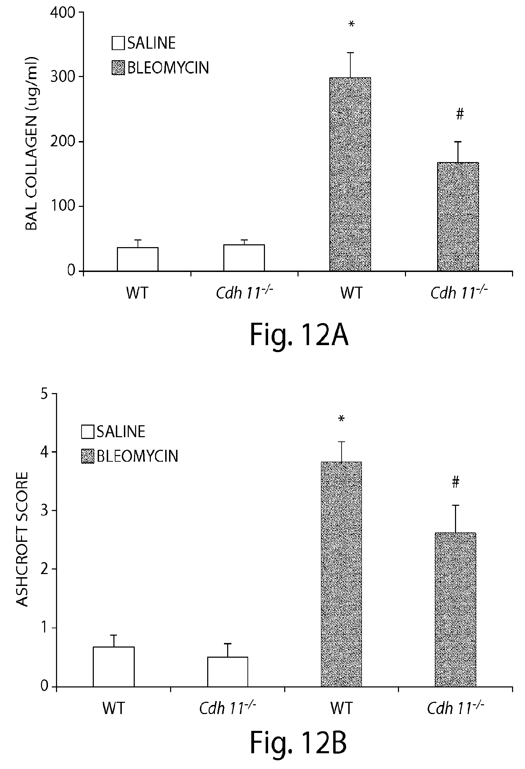

FIG. 12. Quantifiable fibrotic endpoints associated with genetic removal of Cdh11. Fibrosis was quantified by determination of (A) BAL collagen via colorimetric assay and (B) Ashcroft scoring on H&E stained lung sections. Scores were determined on 20 images per mouse lung. n = 6 WT saline, n = 6 Cdh11 ^{-/-} saline, n = 13 WT bleomycin, n = 11 Cdh11 ^{-/-}bleomycin. *P ≤ 0.05 versus WT saline; #P ≤ 0.05 versus WT bleomycin.

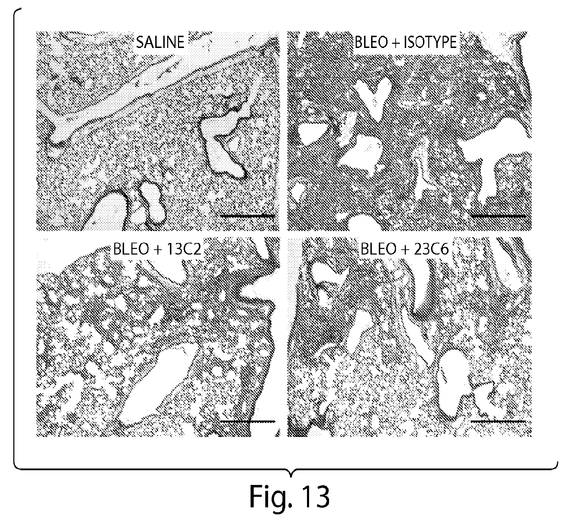

FIG. 13. Cadherin-11 blocking antibodies improves established pulmonary fibrosis. Histopathologic improvement was noted in bleomycin-induced pulmonary fibrosis associated with systemic delivery of cadherin-11 blocking antibodies (23C6 or 13C2) compared with isotype control. Mice received antibody every other day beginning 10 days after bleomycin exposure. Lungs were then taken at day 21 and processed for sectioning and H&E staining. Scale bars = 500 µm. Sections are representative of n = 6 saline, n = 7 Bleo + isotype, n = 8 Bleo + 13C2, and n = 6 Bleo + 23C6.

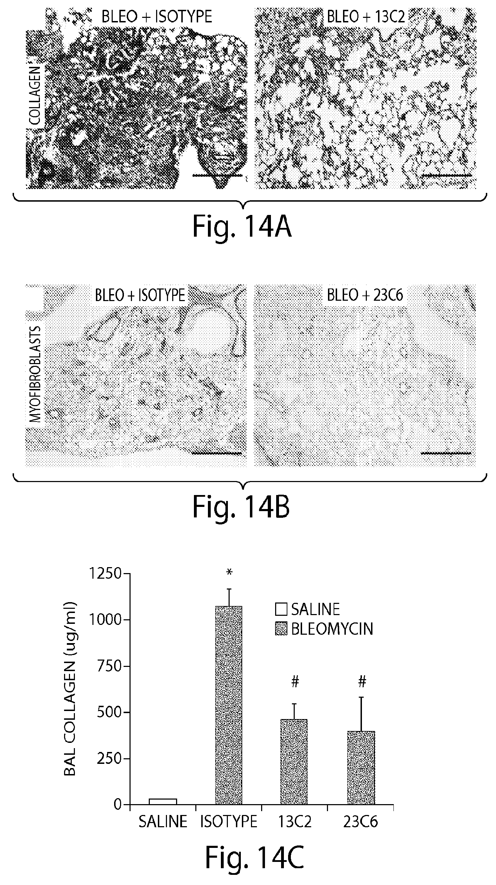

FIG. 14. Cadherin-11 blocking antibody reduces lung collagen and myofibroblasts. Mouse lungs given bleomycin plus systemic antibody were processed and stained for (A) collagen deposition (Masson's Trichrome stain) and (B) myofibroblasts (α-SMA immunohistochemistry). Scale bars = 200 µm. (C) Soluble collagen in BAL fluid quantified with colorimetric assay. n = 6 saline, n = 7 Bleo + isotype, n = 8 Bleo + 13C2, and n = 6 Bleo + 23C6. *P ≤ 0.05 versus saline; #P ≤ 0.05 versus bleomycin + isotype.

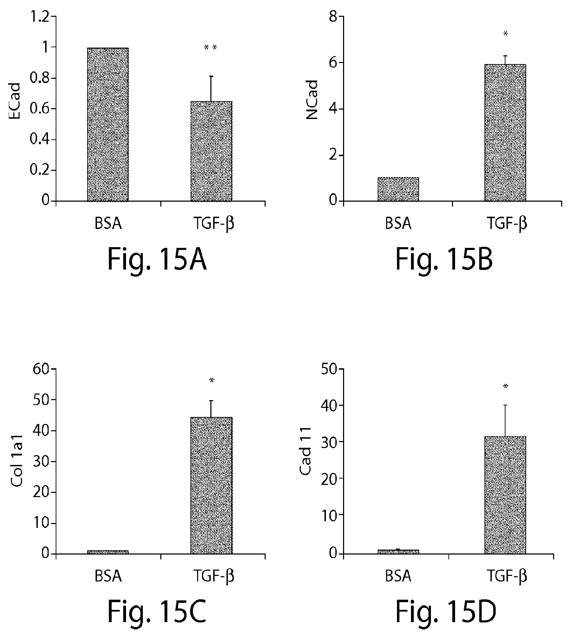

FIG. 15. TGF-β-induced EMT and *Cdh11* expression in A549 lung epithelial cells. A549 lung epithelial cells were stimulated with TGF-β (10 ng/ml) and at 24 hours, RNA was isolated and fold change transcripts (vs. BSA) were determined for (A) E cadherin, (B) N cadherin, (C) α1 pro-collagen, and (D) cadherin-11. *P < 0.05 versus BSA. **P = 0.06 versus BSA. Data representative of 3 separate experiments.

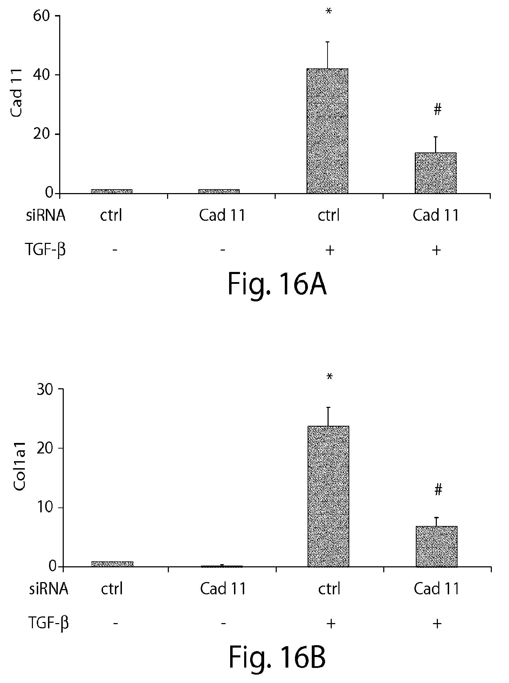

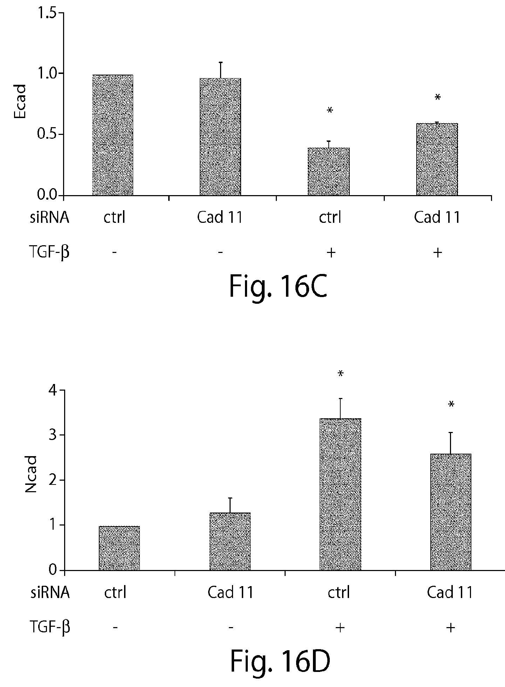

FIG. 16. *Cdh11* knockdown prevents TGF-β-induced EMT of lung epithelial cells. A549 lung epithelial cells were transfected with *Cdh11* or control siRNA, subsequently stimulated with TGF-β and at 24 hours, RNA was isolated and fold change transcripts (vs. BSA + control siRNA) were determined for (A) E cadherin, (B) N cadherin, (C) α1 pro-collagen, and (D) cadherin-11. Data representative of 3 separate experiments.

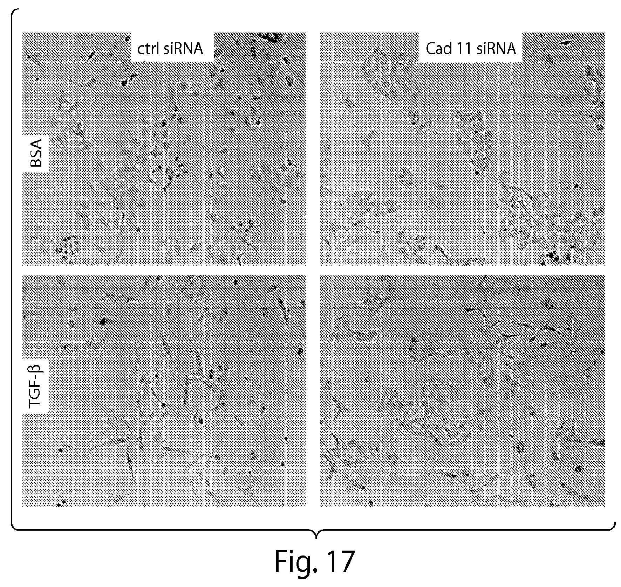

FIG. 17. *Cdh11* knockdown prevents TGF-β-induced transition to mesenchymal morphology in lung epithelial cells. A549 lung epithelial cells were transfected with *Cdh11* or control siRNA and subsequently stimulated with TGF-β. At 24 hours, cell morphology was assessed using phase contrast microscopy.

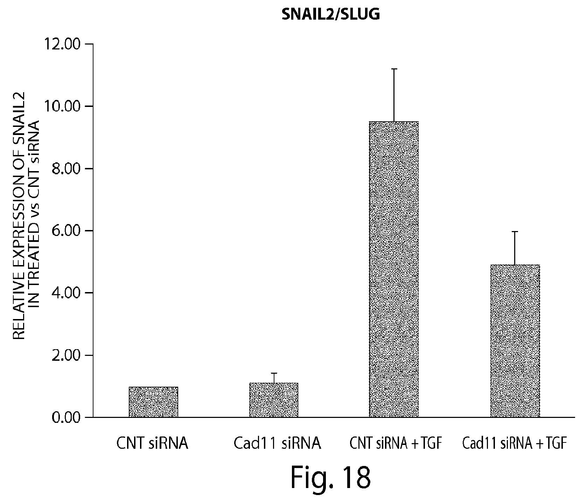

FIG. 18. *Cdh11* knockdown prevents TGF-β-induced SNAIL2 upregulation in lung epithelial cells. A549 lung epithelial cells were transfected with *Cdh11* or control siRNA, subsequently stimulated with TGF-β and at 24 hours, RNA was isolated and fold change transcripts (vs. BSA + control siRNA) were determined for SNAIL2, an EMT transcription factor. Data representative of 3 separate experiments.

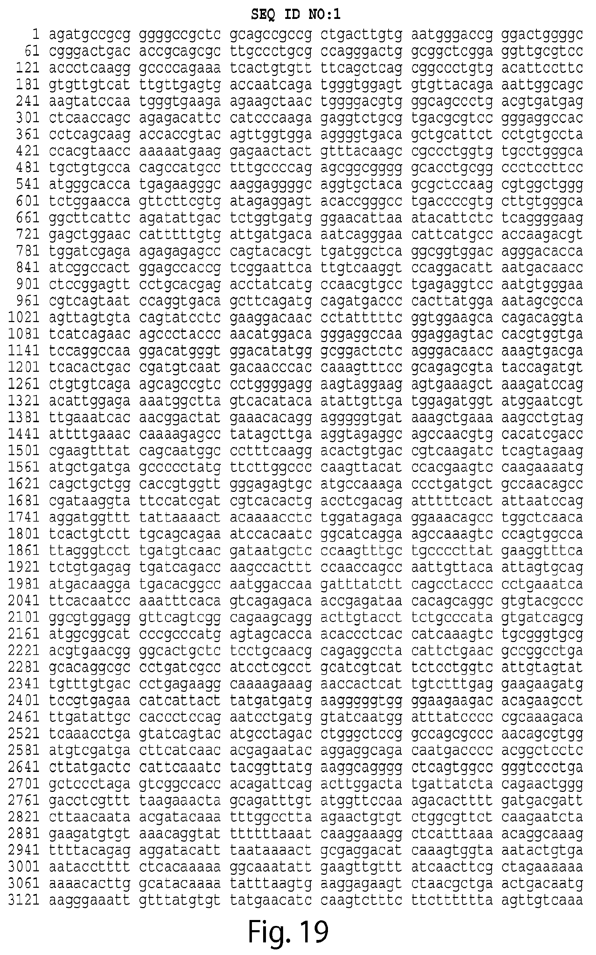

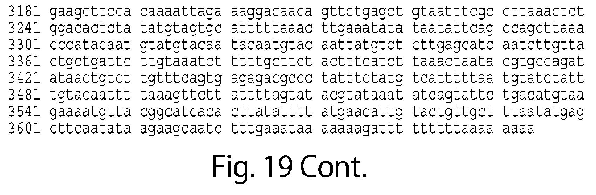

FIG. 19. Nucleotide sequence of human cadherin-11 (SEQ ID NO:1).

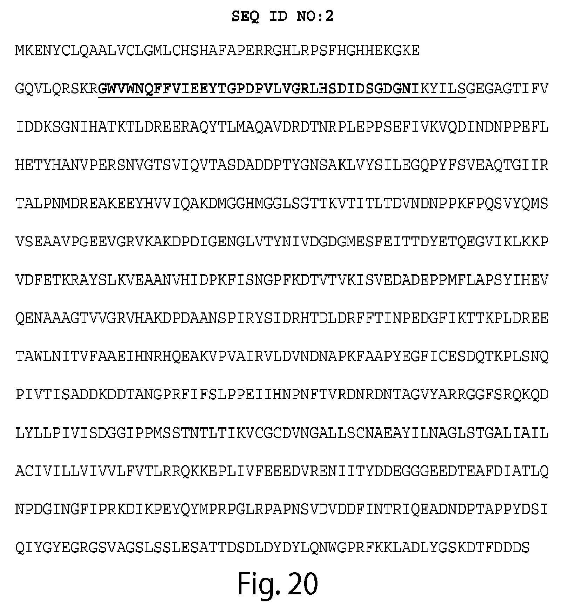

FIG. 20. Amino acid sequence of human cadherin-11 (SEQ ID NO:2).

### DETAILED DESCRIPTION OF INVENTION

The invention is based, in part, on the unexpected finding that cadherin-11 is upregulated in fibrosis including non-dermal fibrosis such as lung or pulmonary fibrosis, and that the lack of cadherin-11 provides some resistance to experimentally-induced fibrosis including non-dermal fibrosis such as lung or pulmonary fibrosis. Based on these findings, the invention contemplates and provides a cadherin-11 antagonist for use in a method of treating fibrosis as defined in the appended claims. The accompanying Examples demonstrate, inter alia, that lung tissue from patients with severe idiopathic pulmonary fibrosis and skin tissue from human patients having scleroderma have increased levels of cadherin-11, and that mice having experimentally-induced lung and/or dermal fibrosis also have increased levels of cadherin-11 in respective tissues. Most strikingly, mice lacking cadherin-11 (i.e., cadherin-11 knock-out mice) have decreased tissue fibrosis in this same experimental model. The Examples further show that cadherin-11 antagonists such as anti-cadherin-11 antibodies are effective in treating (e.g., reducing) fibrosis even after it is established. These data are the first evidence that, inter alia, cadherin-11 is not only a key mediator of fibrosis but also a therapeutic target in the treatment of fibrosis, including lung/pulmonary fibrosis.

### Fibrosis:

Fibrosis refers to the development of excess fibrous connective tissue in an organ or tissue. It is an underlying manifestation of many disease states. Fibrosis can occur in a variety of tissues or organs. These fibrotic conditions include dermal fibrosis (e.g., associated with scleroderma). Dermal fibrosis is fibrosis that manifests itself in the skin (or dermis). Fibrotic conditions also include hypertrophic scars, keloids, burns, Peyronie's disease, and Dupuytren's contractures.

Fibrotic conditions also include non-dermal fibrosis. Non-dermal fibrosis is fibrosis that manifests itself in an organ other than the skin (or dermis). As important example of non-dermal fibrosis is lung (or pulmonary) fibrosis. Lung fibrosis can be associated with interstitial lung disease and diffuse proliferative lung disease. An example of lung fibrosis is idiopathic pulmonary fibrosis (IPF), including IPF with severe airway restriction (referred to herein as severe IPF).

Other examples of non-dermal fibrosis include liver/hepatic fibrosis, ocular fibrosis, fibrosis of the gut, kidney/renal fibrosis, pancreatic fibrosis, vascular fibrosis, cardiac fibrosis, myelofibrosis, and the like.

Some forms of fibrosis are referred to as interstitial fibrosis, and they include dermal or non-dermal interstitial fibrosis.

Fibrosis may be further categorized by its etiology, to the extent such etiology is known. For example, the fibrosis may be associated with or resulting from an infection (e.g., a viral infection or a parasitic infection), or it may be drug-induced fibrosis (e.g., chemotherapy), or it may be associated with or resulting from substance abuse (e.g., alcohol-induced fibrosis), or it may be associated with or resulting from surgery or other invasive procedure, or it may be associated with or resulting from an underlying condition or event (e.g., a myocardial infarction or diabetes), or it may be associated with or resulting from radiation exposure (e.g., radiation treatment for cancer). Examples include liver/hepatic fibrosis associated with alcohol consumption, viral hepatitis and/or schistosomiasis; post-myocardial infarction cardiac fibrosis; kidney/renal fibrosis associated with diabetes; and post-inflammatory kidney/renal fibrosis.

Fibrosis may be transplant-induced, or it may occur independently of transplant (i.e., in a subject that has not undergone a transplant and who is not in need of a transplant). In some embodiments of the invention, the subject has not undergone a kidney (or renal transplant) nor is the subject in need of such a transplant. In still other embodiments, the subject has not undergone a heart transplant nor is the subject in need of such a transplant. In still other embodiments, the subject does not have and/or is not at an elevated risk of developing an inflammatory joint disorder such as rheumatoid arthritis. In still other embodiments, the subjects do not have a cancer and/or are not at an elevated risk of developing cancer.

Various aspects of the disclosure intend to detect (e.g., diagnose), and/or monitor, and/or treat (including prevent) fibrosis in any subject having or susceptible to having fibrosis. The subjects may be human and non-human subjects. Non-human subjects include but are not limited to companion animals (e.g., dogs and cats), agricultural or competitive animals (e.g., cows, horses, etc.).

It is to be understood that the disclosure contemplates methods for detecting fibrosis, based on the presence of abnormally elevated levels of cadherin-11 in cells, organs or tissues. The cells, organs or tissues to be analyzed will depend in part on the type of fibrosis that is suspected of existing or developing or known to exist. For example, the cells, organs or tissues may be skin tissue or skin cells, including fibroblast or fibroblast-like cells resident in skin tissue. As another example, the cells, organs or tissues may be lung tissue or cells such as alveolar macrophages and alveolar epithelial cells. These latter cell types may be obtained in bronchoalveolar lavage. Such methods may be diagnostic in nature (i.e., they may indicate, alone or together with other symptoms or manifestations, that the subject has fibrosis) or they may be prognostic in nature (i.e., they may indicate, alone or with patient history information, that the subject is likely to develop fibrosis). The disclosure further contemplates treating a subject having or at risk of developing fibrosis by administering to such subject a cadherin-11 antagonist, as described in greater detail herein.

### Cadherin-11:

Cadherin-11 is a classical type II cadherin. It comprises a short intracellular domain, a transmembrane domain, and an extracellular domain. The extracellular domain is comprised of 5 subdomains (sometimes themselves referred to as domains), each of which consists of about 110 amino acids. The human and mouse cadherin-11 genes have been isolated and sequenced previously (Suzuki S. et al. Cell Reg 2:261-70, 1991). See also, Genbank Accession No. NM_001797, for the human cadherin-11 cDNA and predicted amino acid sequences (SEQ ID NO: 1 and SEQ ID NO: 2), respectively. Cadherin-11 is also referred to as OB-cadherin, osteoblast cadherin, OSF-4, and CDH11.

The main function of cadherins is to facilitate the adhesion of one cell to another, sometimes similar, cell. Cadherins are involved in cell-to-cell contact and cellular invasion during embryogenesis. In the post-natal tissue, they serve to maintain cell-to-cell contact in epithelial structures. Cadherins likely have other functions beyond cell-to-cell adhesion. For example, as described in the Examples, cadherin-11 is involved in epithelial-to-mesenchymal transition (EMT) during development of fibrosis. Also, as shown in the Examples, cadherin-11 is involved in the differentiation of myofibroblasts from fibroblasts.

### Cadherin-11 Antagonist:

As used herein, the term antagonist refers to any protein, polypeptide, peptide, peptidomimetic, glycoprotein, antibody, antibody fragment, carbohydrate, nucleic acid, organic molecule, inorganic molecule, large molecule, or small molecule that blocks, inhibits, reduces or neutralizes the function, activity and/or expression of another molecule. As used herein, a cadherin-11 antagonist is an agent that blocks, inhibits, reduces or neutralizes the function, activity and/or expression of cadherin-11. As described above, cadherin-11 is involved in cell attachment, interaction and/or migration. Cadherin-11 is known to bind to itself in what is referred to as homophilic or homotypic binding. The cadherin-11 antagonists may interfere with cadherin-11 homotypic binding or heterotypic binding (i.e., binding of cadherin-11 to a counter-receptor that is not cadherin-11). The cadherin-11 antagonist may interfere with cadherin-11 function by reducing the amount of cadherin-11 that is expressed by a cell or by interacting with cadherin-11 (or its counter-receptor) thereby preventing interaction of cadherin-11 with its target. Accordingly, the cadherin-11 antagonist may interfere, in whole or in part, with the transcription of cadherin-11 or with the translation of cadherin-11 (thereby interfering with cadherin-11 expression), or it may interfere with the ability of cadherin-11 to bind to another cadherin-11 or to another cadherin-11 counter-receptor. The cadherin-11 antagonist may reduce cadherin-11 function or activity by about 10%, 20%, 30%, 40%, 50%, 60%, 70%, 80%, 90% or 100%, relative to a control such as PBS. It will be understood that the cadherin-11 antagonist may be used in an amount that reduces cadherin-11 function or activity by about these amounts. It will further be understood that some cadherin-11 antagonists are preferably used in vitro while others are more suitable for the in vivo methods provided herein.

Some cadherin-11 antagonists bind to the extracellular domain of cadherin-11, some bind to particular regions of the extracellular domain of cadherin-11. As discussed herein, the cadherin-11 extracellular domain is comprised of five (5) subdomains each approximately about 110 amino acids in size. (See, for example, U.S. Patent No. 7589074 and Yagi et al. Genes and Development, 14:1169-1180, 2000.) The invention contemplates the use of cadherin-11 antagonists that bind to cadherin-11 EC1 or to a fragment of cadherin-11 EC1 (e.g., a fragment that comprises about the first 33 through to the first 37 amino acids of EC1), or to a fragment of cadherin-11 that comprises EC1 (or the first 33-37 amino acids of EC1). In some embodiments, the antagonist binds to a region of EC1 having an amino acid sequence of GWVWN QFFVI EEYTG PDPVL VGRLH SDIDS GDGN (SEQ ID NO:3, the first 34 amino acids of EC1). Alternatively or additionally, the antagonist may comprise some or all of this amino acid sequence.

The cadherin-11 antagonist may be a peptide or protein, or it may be a nucleic acid, or it may be a organic or inorganic small molecule. The antagonists may be naturally occurring or non-naturally occurring. They may be isolated from a naturally occurring source or they may be synthesized in vitro.

The cadherin-11 antagonists may be conjugated to another agent such as an imaging agent or a cytotoxic agent. Imaging agents may be used to visualize cadherin-11 expression in vitro (e.g., for immunohistochemical analysis) or in vivo (e.g., for body imaging). Examples include radionuclides, contrast agents, and particulates routinely used in medical imaging. Cytotoxic agents are agents that are toxic to cells. Examples include chemotherapeutic agents, toxins, and the like. The use of these agents conjugated to a cadherin-11 antagonist will target such agents to fibrotic tissue and cells. In these instances, therapeutic benefit may be provided by a combination of the cadherin-11 antagonist which interferes with the ability of cadherin-11 to bind to a counter-receptor and the cytotoxic agent which is directly toxic to cells.

### Cadherins-11 Blinding Peptides:

Cadherin-11 antagonists that are peptide or protein in nature include (1) a full length cadherin-11 protein, (2) a fragment of the full length protein, wherein the fragment comprises the transmembrane domain of cadherin-11 or a fragment of the extracellular domain including for example a fragment comprising or consisting of EC1 (e.g., a fragment that comprises EC1, a fragment that comprises EC1 and EC2, a fragment that comprises EC1-EC3, a fragment that comprises EC1-EC4, a fragment that comprises EC1-EC5, a fragment that comprises EC1 and EC3, a fragment that comprises EC1 and EC4, a fragment that comprises EC1 and EC5), (3) a fragment of the full length protein, wherein the fragment comprises one or more of cadherin-11 extracellular subdomains (e.g., EC1, EC2, EC3, EC4, or EC5 of the 5 extracellular subdomains of cadherin-11, or any combination thereof), (4) fusion proteins that comprise full length cadherin-11 or a fragment thereof, and (5) antibodies and fragments thereof. In important embodiments, the cadherin-11 antagonist binds to and/or comprises the EC1 domain of cadherin-11 or a fragment thereof (such as SEQ ID NO:3 provided herein). Cadherin-11 antagonists that are peptide or protein in nature preferably will bind preferentially (or selectively) to cadherin-11. Preferential (or selective) binding to cadherin-11 means that the peptide or protein binds with greater affinity to cadherin-11 than to another protein. In some instances, the peptide or protein binds to cadherin-11 with an affinity that is about 2-fold more, about 3-fold more, about 4-fold more, about 5-fold more, about 10-fold more, about 25-fold more, about 50-fold more, about 100-fold more, about 1000-fold more, or more than its affinity for a protein that is not cadherin-11 or for any other moiety. Such differences in affinity are preferably manifest under physiological conditions as occur in vivo. In some embodiments, the cadherin-11 binding peptides bind to EC1 of cadherin-11, and optionally to the first 33-37 amino acids, including the first 33, first 34, first 35, first 36, or first 37 amino acids of EC1 of cadherin-11, as shown in SEQ ID NO:2 provided herein. Binding to this region of cadherin-11 can be determined through competitive binding assays using other binding agents known to bind to this region of cadherin-11 such as those described in WO2009/089062. The afore-mentioned antagonists are collectively referred to as cadherin-11 binding peptides. Cadherin-11 binding peptides may be harvested and isolated from naturally occurring sources or they may be synthesized and screened for their ability to bind to cadherin-11.

As used herein with respect to peptides and proteins, the term "isolated" means separated from its native environment in sufficiently pure form so that it can be manipulated or used for any one of the purposes of the invention.

Binding peptides can also be derived from sources other than antibody technology. For example, binding peptides can be provided by degenerate peptide libraries which can be readily prepared in solution, in immobilized form, as bacterial flagella peptide display libraries or as phage display libraries. Combinatorial libraries also can be synthesized of peptides containing one or more amino acids. Libraries can also be made that are comprised of peptides and non-peptide synthetic moieties.

Cadherin-11, or a fragment thereof, also can be used to isolate other cadherin-11 binding peptides or partners. Isolation of binding partners may be performed according to well-known methods. For example, cadherin-11 or a fragment thereof (e.g., an extracellular fragment) can be attached to a substrate, and then a putative cadherin-11 binding peptide may be applied to the substrate. If a cadherin-11 binding peptide is present, it will bind to the substrate-bound cadherin-11, and it can then be isolated and further analyzed.

### Full-Length Cadherin-11 and Cadherin-11 Fragments:

Based on the known nucleotide and amino acid sequence of cadherin-11, suitable fragments of cadherin-11 may be identified and generated using conventional technology. Reference may be made to U.S. Patent Nos. 5597725, 5639634, 5646250, 6787136, 6946768, 7488478, and 7589074, and PCT Patent Publication Nos. WO 93/21302 and WO2009/089062, relating to cadherin-11 nucleotide and amino acid sequences and fragments.

Examples of suitable fragments include those that consist of or comprise amino acids 1-40, 1-39, 1-38, 1-37, 1-36, 1-35, 1-34, 1-33, 1-32, 1-31 or 1-30 of cadherin EC1 or those that consist of or comprise amino acids 15-34, 15-35, 15-36, 15-37, 15-38, 15-39, or 15-40 of cadherin EC1. The first 40 amino acids of EC1 are underlined and the first 35 amino acids of EC1 are bolded in SEQ ID NO:2 as provided herein. Examples of suitable fragments are also provided in WO2009/089062 (represented by the amino acid sequences of SEQ ID NOs: 3, 10, 12, and 13, and also described in US 2009/0253200). Other fragments may comprise amino acids 1-160, or 1-259, or 1-269 of SEQ ID NO:2, and optionally they may lack amino acids 1-53 of SEQ ID NO:2 which represents the leader and pro-region of human cadherin-11.

Cadherin-11 binding peptides may also be variants of full-length cadherin-11 or cadherin-11 fragments. Such variants may differ from cadherin-11 amino acid sequence by a degree. For example, variants may be about 80%, about 85%, about 90%, about 91%, about 92%, about 93%, about 94%, about 95%, about 96%, about 97%, about 98%, or about 99% identical to full length cadherin-11 or to a cadherin-11 fragment. Variants may comprise a cadherin-11 fragment and additional flanking constituents at the amino and/or carboxy end of the fragment. Such constituents may be amino acid in nature. In all instances, the variants bind to cadherin-11 and interfere with cadherin-11 function or activity.

Cadherin-11 binding peptides may be at least 10, 20, 30, 40, 50, 60, 70, 80, 90, 100, 110, amino acids in length or longer. For example, they may be about or at least 220, 330, 440, 550 amino acids in length.

In some important embodiments, the cadherin-11 inhibitory agent is a functionally equivalent peptide analog of cadherin-11. As used herein, the term functionally equivalent peptide analog refers to a peptide analog that is capable of inhibiting the binding of cadherin-11 to, for example, itself. Functionally equivalent peptide analogs of cadherin-11 are identified, for example, using in vitro adhesion assays that measure the ability of the peptide analog to inhibit cadherin-11-mediated adhesion either between cells expressing cadherin-11 or between isolated cadherin-11 proteins, or some combination thereof. Accordingly, exemplary functionally equivalent peptide analogs of cadherin-11 include analogs of full length cadherin-11 or a cadherin-11 fragment that for example comprises conservative amino acid substitutions relative to the wild-type sequence.

Still other cadherin-11 binding peptides are provided in PCT Published Application Nos. WO99/57149, WO2004/048411 and WO2009/089062.

### Cadherin-11 Fusion Proteins:

The cadherin-11 binding peptide can be a fusion protein. A fusion protein, as used herein, is a protein that contains peptide regions from at least two different proteins. For example, a cadherin-11 fusion protein contains amino acid sequence from cadherin-11 and at least one non-cadherin-11 protein. Such fusion proteins can be formed by fusing, usually at the nucleotide level, coding sequence from cadherin-11 to coding sequence from a non-cadherin-11 protein. Examples of cadherin-11 fusion proteins include cadherin-11 GST fusion protein, cadherin-11 Fc fusion protein, cadherin-11 beta-galactosidase fusion protein, cadherin-11 poly-His fusion protein, and cadherin-11 GFP fusion protein. Fc fusion proteins may comprise regions of the Ig constant domain, including without limitation the hinge region, the CH1 domain, the CH2 domain, and/or CH3 domain, optionally conjugated to the cadherin-11 fragment via the hinge domain. The Fc portion may derive from human antibodies or non-human antibodies. The antibodies may be IgG1 or IgG2, although they are not so limited. Methods of making Fc fusion proteins are known in the art and are described at least in EP0464533.

In some embodiments, the cadherin-11 fusion proteins comprise the entire extracellular domain of cadherin-11. In some embodiments, the cadherin-11 fusion protein comprises one or more extracellular subdomains of cadherin-11, such as EC1. Examples include fusion proteins comprising EC1, EC1/2, EC1-3, EC1-4, EC1/3, EC1/4, and EC1/5, or fragments of EC1. In important embodiments, the fusion protein binds to the EC1 domain of cadherin-11. Examples of cadherin-11 fusion proteins include cadherin-11-EC1-Fc fusion protein (comprising the EC1 domain of cadherin-11), cadherin-11-EC1/2-Fc fusion protein (comprising the EC1 and EC2 domains of cadherin-11), and cadherin-11-EC1-5-Fc fusion protein (comprising the EC1, EC2, EC3, EC4, and EC5 domains of cadherin-11). Some fusion proteins may comprise the first 40, first 39, first 38, first 37, first 36, first 35, or first 34 amino acids of the EC1 domain of cadherin-11, as described in WO 2009/089062.

Methods of synthesis of cadherin-11 fusion proteins can be found at least in U.S. Patent Nos. 5597725, 5639634, 5646250, 6787136, 6946768, 7488478, and 7589074 and PCT Patent Publication No. WO 93/21302 and WO2009/089062 (see for example SEQ ID NOs: 6 and 7, the nucleotide and amino acid sequences of a human cadherin-11-EC1-hIgG2-Fc fusion protein).

### Cadherin-11 Antibodies and Antibody Fragments:

Cadherin-11 antagonists that are cadherin-11 binding peptides may be antibodies or antigen-binding antibody fragments. The antibodies may be monoclonal antibodies or polyclonal antibodies. They may be chimeric antibodies including humanized antibodies. They may be four chain antibodies comprised of two heavy and two light chains, or they may be two chain antibodies such as those comprised of two heavy chains (such as camelid antibodies) or those comprised of a single heavy chain linked to a single light chain (such as a single chain Fvs). They can be of any type (e.g., IgG, IgE, IgM, IgD, IgA and IgY), class (e.g., IgG1, IgG2, IgG3, IgG4, IgA1 and IgA2) or subclass. As discussed below, these various antibody forms can be prepared according to conventional methodology. The antibodies and antibody fragments may be naturally occurring or non-naturally occurring including for example recombinantly produced antibodies and fragments.

Significantly, as is well-known in the art, only a small portion of an antibody molecule, the paratope, is involved in the binding of the antibody to its epitope (see, in general, Clark, W.R. (1986) The Experimental Foundations of Modern Immunology Wiley & Sons, Inc., New York; Roitt, I. (1991) Essential Immunology, 7th Ed., Blackwell Scientific Publications, Oxford). The pFc' and Fc regions, for example, are effectors of the complement cascade but are not involved in antigen binding. An antibody from which the pFc' region has been enzymatically cleaved, or which has been produced without the pFc' region, designated an F(ab')₂ fragment, retains both of the antigen binding sites of an intact antibody. Similarly, an antibody from which the Fc region has been enzymatically cleaved, or which has been produced without the Fc region, designated an Fab fragment, retains one of the antigen binding sites of an intact antibody molecule. Proceeding further, Fab fragments consist of a covalently bound antibody light chain and a portion of the antibody heavy chain denoted Fd. The Fd fragments are the major determinant of antibody specificity (a single Fd fragment may be associated with up to ten different light chains without altering antibody specificity) and Fd fragments retain epitope-binding ability in isolation.

The terms Fab, Fab', Fc, Fd, pFc', F(ab')₂, Fv, and dAb are employed with either standard immunological meanings [Klein, Immunology (John Wiley, New York, NY, 1982); Clark, W.R. (1986) The Experimental Foundation of Modern Immunology (Wiley & Sons, Inc., New York); Roitt, I. (1991) Essential Immunology, 7th Ed., (Blackwell Scientific Publications, Oxford)]. Well-known functionally active antibody fragments include but are not limited to F(ab')₂, Fab, Fv and Fd fragments of antibodies. These fragments which lack the Fc fragment of intact antibody, clear more rapidly from the circulation, and may have less non-specific tissue binding than an intact antibody (Wahl et al., J. Nucl. Med. 24:316-325 (1983)). For example, single-chain antibodies can be constructed in accordance with the methods described in U.S. Patent No. 4,946,778 to Ladner et al. Such single-chain antibodies include the variable regions of the light and heavy chains joined by a flexible linker moiety. Methods for obtaining a single domain antibody ("Fd") which comprises an isolated variable heavy chain single domain, also have been reported (see, for example, Ward et al., Nature 341:644-646 (1989), disclosing a method of screening to identify an antibody heavy chain variable region (V_{H} single domain antibody) with sufficient affinity for its target epitope to bind thereto in isolated form). Methods for making recombinant Fv fragments based on known antibody heavy chain and light chain variable region sequences are known in the art and have been described, e.g., Moore et al., US Patent No. 4,462,334. Other references describing the use and generation of antibody fragments include e.g., Fab fragments (Tijssen, Practice and Theory of Enzyme Immunoassays (Elsevieer, Amsterdam, 1985)), Fv fragments (Hochman et al., Biochemistry 12: 1130 (1973); Sharon et al., Biochemistry 15: 1591 (1976); Ehrilch et al., U.S. Patent No. 4,355,023) and portions of antibody molecules (Audilore-Hargreaves, U.S. patent No. 4,470,925). Thus, those skilled in the art may construct antibody fragments from various portions of intact antibodies without destroying the specificity of the antibodies.

Within the antigen-binding portion of an antibody, as is well-known in the art, there are complementarity determining regions (CDRs), which directly interact with the epitope of the antigen, and framework regions (FRs), which maintain the tertiary structure of the paratope (see, in general, Clark, 1986; Roitt, 1991). In both the heavy chain Fd fragment and the light chain of IgG immunoglobulins, there are four framework regions (FR1 through FR4) separated respectively by three complementarity determining regions (CDR1 through CDR3). The CDRs, and in particular the CDR3 regions, and more particularly the heavy chain CDR3, are largely responsible for antibody specificity.

It is now well-established in the art that the non-CDR regions of a mammalian antibody may be replaced with similar regions of conspecific or heterospecific antibodies while retaining the epitopic specificity of the original antibody. This is most clearly manifested in the development and use of "humanized" antibodies in which non-human CDRs are covalently joined to human FR and/or Fc/pFc' regions to produce a functional antibody. Thus, for example, PCT International Publication No. WO 92/04381 and published European Patent Application No. EP 0239400 teach the production and use of humanized murine antibodies in which at least a portion of the murine FR regions have been replaced by FR regions of human origin. Such antibodies, including fragments of intact antibodies with antigen-binding ability, are often referred to as "chimeric" antibodies. There are entities in the United States which will synthesize humanized antibodies from specific murine antibody regions commercially, such as Protein Design Labs (Mountain View California), Abgenix, and Medarex.

Thus, as will be apparent to one of ordinary skill in the art, the present disclosure also provides for F(ab')₂, Fab, Fv and Fd fragments; chimeric antibodies in which the Fc and/or FR and/or CDR1 and/or CDR2 and/or light chain CDR3 regions have been replaced by homologous human or non-human sequences; chimeric F(ab')₂ fragment antibodies in which the FR and/or CDR1 and/or CDR2 and/or light chain CDR3 regions have been replaced by homologous human or non-human sequences; chimeric Fab fragment antibodies in which the FR and/or CDR1 and/or CDR2 and/or light chain CDR3 regions have been replaced by homologous human or non-human sequences; and chimeric Fd fragment antibodies in which the FR and/or CDR1 and/or CDR2 regions have been replaced by homologous human or non-human sequences. The present disclosure also includes single chain antibodies.

In addition, human monoclonal antibodies may be made by any of the methods known in the art, such as those disclosed in US Patent No. 5,567,610, issued to Borrebaeck et al., US Patent No. 565,354, issued to Ostberg, US Patent No. 5,571,893, issued to Baker et al, Kozber, J. Immunol. 133: 3001 (1984), Brodeur, et al., Monoclonal Antibody Production Techniques and Applications, p. 51-63 (Marcel Dekker, Inc, new York, 1987), and Boerner et al., J. Immunol., 147: 86-95 (1991). In addition to the conventional methods for preparing human monoclonal antibodies, such antibodies may also be prepared by immunizing transgenic animals that are capable of producing human antibodies (e.g., Jakobovits et al., PNAS USA, 90: 2551 (1993), Jakobovits et al., Nature, 362: 255-258 (1993), Bruggermann et al., Year in Immunol., 7:33 (1993) and US Patent No. 5,569,825 issued to Lonberg).

Exemplary cadherin-11 antibodies and methods for making such antibodies are described in U.S. Patent Nos. 5597725, 5639634, 5646250, 6787136, 6946768, 7488478, and 7589074, and PCT Patent Publication No. WO 93/21302 and WO2009/089062. Examples of cadherin-11 antibodies include 23C6, 13C2, 27F3, 5F82 (commercially available from Lifespan Science), H1M1 antibody (cadherin-11 EC1 specific antibody produced by hybridoma H1M1 having ATCC Accession No. PTA-9699), H14 antibody (cadherin-11 EC1 specific antibody produced by hybridoma H14 having ATCC Accession No. PTA-9701), BM5096/1A6 (commercially available from Acris Antibodies GmbH), 283416 (commercially available from R&D Systems), and MAB2014 (commercially available from Millipore). Examples of cadherin-11 antibody fragments include the Fab fragment of antibodies 23C6, 13C2, 27F3, 5F82, H1M1 antibody, H14 antibody, BM5096/1A6, 283416, and MAB2014. The antibodies or antibody fragments may comprise one or more CDRs from known antibodies such as the H1M1 or H14 antibodies, as described in US 2009/0253200.

Antibodies and antibody fragments that bind to the EC1 domain of cadherin-11 are described in US 2009/0253200 and WO2009/089062.

Cadherin-11 antibodies may also be bispecific or bifunctional antibodies capable of binding to two different epitopes by virtue of their different antigen-binding sites.

Still other cadherin-11 antibodies are camelid antibodies as described in PCT Publication No. WO 94/04678 and U.S. Patent Publication No. 20080124324, and their derivatives in the form of camelid nanobodies as in U.S. Patent No. 5759808. Camelid antibodies and camelid nanobodies are commercially available from sources such as Ablynx (Belgium). It is to be understood that the cadherin-11 camelid antibodies can be humanized in a manner similar to that described herein for other antibody types.

### Cadherin-11 Nucleic Acid Antagonists:

A cadherin-11 antagonist may also be a nucleic acid. These antagonists include nucleic acids that (1) encode a cadherin-11 polypeptide or a fragment thereof; (2) are cadherin-11 antisense molecules which inhibit the transcription or translation of the foregoing nucleic acid molecules; (3) are cadherin-11 inhibitory RNA (e.g., siRNA or shRNA); (4) are cadherin-11 ribozymes; (5) aptamers that are nucleic acid in nature but bind to the cadherin-11 as would binding peptides thereby interfering with the binding of cadherin-11 to another cadherin-11 or to another cadherin-11 counter-receptor. In some embodiments, a cadherin-11 antagonist that is a nucleic acid (1) hybridizes under stringent conditions to a nucleic acid having a sequence of SEQ ID NO: 1, and (2) codes for a cadherin-11 polypeptide or a fragment thereof that is capable of binding specifically to cadherin-11.

### Cadherin-11 Encoding Nucleic Acids:

Cadherin-11 antagonists include nucleic acids that encode cadherin-11 and fragments of cadherin-11. The cadherin-11 full length nucleotide sequence is provided as SEQ ID NO: 1. Nucleic acids comprising a nucleotide sequence of SEQ ID NO:1 may be used as antagonists, as an example. The cadherin-11 antagonists of the invention also include homologs and alleles of a nucleic acid molecule comprising a sequence of SEQ ID NO: 1.

The cadherin-11 nucleic acid antagonists, may encode polypeptides which are soluble cadherin-11 polypeptides or membrane-bound polypeptides, or cadherin-11 fragments such as fragments that consist of or comprise EC1 or a fragment thereof (e.g., the first 33-37 amino acids of EC1). The soluble cadherin-11 polypeptides lack a transmembrane domain and, optimally, contain further amino acids which render the polypeptide soluble (e.g., fusion proteins, containing all or part of cadherin-11, which inhibit the binding of cadherin-11 to another cadherin-11). Cadherin-11 fragments which are membrane-bound (or membrane associated) preferably contain a transmembrane domain.

Cadherin-11 nucleic acid antagonists further embrace nucleic acid molecules which code for a cadherin-11 protein having the amino acid sequence of SEQ ID NO: 2 (or SEQ ID NO:3, for example), but which may differ from the sequence of SEQ ID NO: 1 due to the degeneracy of the genetic code.

Certain cadherin-11 nucleic acid antagonists can be identified by conventional techniques, e.g., by identifying nucleic acid sequences which code for cadherin-11 and which hybridize to a nucleic acid molecule having the sequence of SEQ ID NO: 1 under stringent conditions. The term "stringent conditions," as used herein, refers to parameters with which the art is familiar. More specifically, stringent conditions, as used herein, refer to hybridization at 65C in hybridization buffer (3.5 x SSC, 0.02 % formamide, 0.02 % polyvinyl pyrolidone, 0.02 % bovine serum albumin, 2.5 mM NaH₂PO₄ (pH 7), 0.5 % SDS, 2 mM EDTA). SSC is 0.15 M sodium chloride/0.15 M sodium citrate, pH 7; SDS is sodium dodecyl sulphate; and EDTA is ethylene-diamine-tetra-acetic acid. After hybridization, the membrane to which the DNA is transferred is washed at 2x SSC at room temperature and then at 0.1x SSC/0.1x SDS at 65C.

There are other conditions, reagents, and so forth which can be used, which result in a similar degree of stringency. The skilled artisan will be familiar with such conditions and, thus, they are not given here. It will be understood, however, that the skilled artisan will be able to manipulate the conditions in a manner to permit the clear identification of homologs and alleles of the nucleic acid molecules of the invention. The skilled artisan also is familiar with the methodology for screening cells and libraries for the expression of further nucleic acids molecules which can be isolated and sequenced. In screening for cadherin-11 sequences for example, a Southern blot may be performed using the foregoing conditions, together with a radioactive probe. After washing the membrane to which the DNA is finally transferred, the membrane can be placed against x-ray film to detect the radioactive signal.

In general, cadherin-11 homologs and alleles typically will share at least 70% nucleotide identity with SEQ. ID. NO: 1; and in some instances, will share at least 75% nucleotide identity; and in still other instances, will share at least 80% nucleotide identity. Watson-Crick complements of the foregoing nucleic acids are also embraced by the disclosure. The preferred cadherin-11 homologs have at least 85% sequence homology to SEQ. ID. NO: 1. More preferably the cadherin-11 homologs have at least 90% and most preferably at least 95% sequence homology to SEQ. ID. NO: 1. The homology can be calculated using various, publicly available software tools developed by NCBI (Bethesda, Maryland) that can be obtained through the internet. Exemplary tools include the BLAST system available at the NCBI website. Pairwise and ClustalW alignments (BLOSUM30 matrix setting) as well as Kyte-Doolittle hydropathic analysis can be obtained using the MacVector sequence analysis software (Oxford Molecular Group).

The disclosure also includes degenerate nucleic acids which include alternative codons to those present in the naturally occurring nucleic acid that encodes, for example, the human cadherin-11 polypeptide. As is well known in the art, and as an example, serine residues are encoded by the codons TCA, AGT, TCC, TCG, TCT and AGC. Each of the six codons is equivalent for the purposes of encoding a serine residue. Thus, it will be apparent to one of ordinary skill in the art that any of the serine-encoding nucleotide codons may be employed to direct the protein synthesis apparatus, in vitro or in vivo, to incorporate a serine residue. Similarly, nucleotide sequence triplets which encode other amino acid residues include, but are not limited to, CCA, CCC, CCG and CCT (proline codons); CGA, CGC, CGG, CGT, AGA and AGG (arginine codons); ACA, ACC, ACG and ACT (threonine codons); AAC and AAT (asparagine codons); and ATA, ATC and ATT (isoleucine codons). Other amino acid residues may be encoded similarly by multiple nucleotide sequences.

As used herein with respect to nucleic acids, the term "isolated" means: (i) amplified *in vitro* by, for example, polymerase chain reaction (PCR); (ii) recombinantly produced by cloning; (iii) purified, as by cleavage and gel separation; or (iv) synthesized by, for example, chemical synthesis. An isolated nucleic acid is one which is readily manipulable by recombinant DNA techniques well known in the art. Thus, a nucleotide sequence contained in a vector in which 5' and 3' restriction sites are known or for which polymerase chain reaction (PCR) primer sequences have been disclosed is considered isolated but a nucleic acid sequence existing in its native state in its natural host is not. An isolated nucleic acid may be substantially purified, but need not be. For example, a nucleic acid that is isolated within a cloning or expression vector is not pure in that it may comprise only a tiny percentage of the material in the cell in which it resides. Such a nucleic acid is isolated, however, as the term is used herein because it is readily manipulable by standard techniques known to those of ordinary skill in the art.

The cadherin-11 nucleic acid antagonist, in one embodiment, is operably linked to a gene expression sequence which directs the expression of the cadherin-11 nucleic acid antagonist within a cell such as a eukaryotic cell. The "gene expression sequence" is any regulatory nucleotide sequence, such as a promoter sequence or promoter-enhancer combination which facilitates the efficient transcription and translation of the cadherin-11 nucleic acid antagonist to which it is operably linked. The gene expression sequence may, for example, be a mammalian or viral promoter, such as a constitutive or inducible promoter. Constitutive mammalian promoters include, but are not limited to, the promoters for the following genes: hypoxanthine phosphoribosyl transferase (HPTR), adenosine deaminase, pyruvate kinase, beta-actin promoter and other constitutive promoters. Exemplary viral promoters which function constitutively in eukaryotic cells include, for example, promoters from the simian virus, papilloma virus, adenovirus, human immunodeficiency virus (HIV), Rous sarcoma virus, cytomegalovirus, the long terminal repeats (LTR) of moloney leukemia virus and other retroviruses, and the thymidine kinase promoter of herpes simplex virus. Other constitutive promoters are known to those of ordinary skill in the art. The promoters useful as gene expression sequences of the disclosure also include inducible promoters. Inducible promoters are expressed in the presence of an inducing agent. For example, the metallothionein promoter is induced to promote transcription and translation in the presence of certain metal ions. Other inducible promoters are known to those of ordinary skill in the art.

In general, the gene expression sequence shall include, as necessary, 5' non-transcribing and 5' non-translating sequences involved with the initiation of transcription and translation, respectively, such as a TATA box, capping sequence, CAAT sequence, and the like. Especially, such 5' non-transcribing sequences will include a promoter region which includes a promoter sequence for transcriptional control of the operably joined cadherin-11 nucleic acid antagonist. The gene expression sequences optionally includes enhancer sequences or upstream activator sequences as desired.

Cadherin-11 nucleic acid antagonist may be used in both in vivo and in vitro methods. Nucleic acid molecules may be introduced into a cell in vitro, followed by the transfer of the cell to the site of fibrosis. The cell into which the nucleic acid molecule is introduced may be harvested from the site of fibrosis (e.g., a fibroblast) or it may be a cell which is not normally present at the site of inflammation. A sequence which permits expression of the nucleic acid in a particular tissue (or cell), such as for example the lung, is one which is selectively transcriptionally active in the tissue (or cell) and thereby causes the expression of the nucleic acid in the tissue (or cell). Those of ordinary skill in the art will be able to easily identify alternative promoters that are capable of expressing such a nucleic acid molecule in lung tissue, liver tissue, renal tissue, and the like, as mentioned herein. Alternatively, a cell transduced with the cadherin-11 nucleic acid antagonist may be cultured in vitro in order to produce a cadherin-11 protein antagonist or it may be used in in vitro screening assays. For example, the gene expression sequence may be used to express cadherin-11 in a cell which does not inherently express cadherin-11.

The nucleic acid molecule sequences and the gene expression sequence are said to be "operably linked" when they are covalently linked in such a way as to place the transcription and/or translation of the nucleic acid antagonist (e.g., a cadherin-11 coding sequence) under the influence or control of the gene expression sequence. If it is desired that nucleic acid molecule be translated into a functional protein, two DNA sequences are said to be operably linked if induction of a promoter in the 5' gene expression sequence results in the transcription of the nucleic acid molecule and if the nature of the linkage between the two DNA sequences does not (1) result in the introduction of a frame-shift mutation, (2) interfere with the ability of the promoter region to direct the transcription of the nucleic acid molecule, or (3) interfere with the ability of the corresponding RNA transcript to be translated into a polypeptide. Thus, a gene expression sequence would be operably linked to a nucleic acid molecule if the gene expression sequence were capable of effecting transcription of that nucleic acid molecule such that the resulting transcript might be translated into the desired polypeptide.

### Cadherin-11 siRNA:

The invention contemplates the use of RNA interference agents such as siRNA and shRNA as cadherin-11 antagonists. siRNA are RNA molecules capable of causing interference and thus post-transcriptional silencing of specific genes in cells, including mammalian cells. siRNA comprise a double stranded region that is typically about 5-50 base pairs, more typically 10-40 base pairs, and even more typically 15-30 base pairs in length. The siRNA may be 20-50, 25-50 or 30-40 base pairs in length. These siRNA may be digested by the RNase III Dicer to yield smaller siRNA in the range of 19-28 base pairs, including 19 base pairs, 21 base pairs, 23 base pairs, 25 base pairs, and 27 base pairs in length. It is known that siRNA in this size range can be incorporated into and acted upon by the enzyme complex called RNA-Induced Silencing Complex (RISC), with a net result of target RNA degradation and/or inhibition of any protein translation therefrom. In a similar manner, double-stranded RNAs with other regulatory functions such as microRNAs (miRNA) can also be used. Reference can be made to Bass, Nature 411: 428-29 (2001); Elbashir et al., Nature 411: 494-98 (2001); Fire et al., Nature 391: 806-11 (1998); WO 01/75164, and US Patents 6506559, 7056704, 7078196, 7432250, for greater detail on siRNA as well as methods of making siRNA. siRNA to cadherin-11 are commercially available from sources such as Dharmacon.

siRNA forms such as the R- and L-form will have overhangs on one or both ends. As discussed herein, an R-form siRNA has a 3' overhang on its antisense strand. It may be blunted on its other end and/or it may have a 3' overhang on its other end, including an overhang comprising DNA residues. Alternatively, an L-form siRNA has a 3' overhang on its sense strand. It may be blunted on its other end and/or it may have a 3' overhang on its other end, including an overhang comprising DNA residues.

siRNA may be comprised of ribonucleotides or a combination of ribonucleotides and deoxyribonucleotides, including in some instances modified versions of one or both. For example, ribonucleotides containing a non-naturally occurring base (instead of a naturally occurring base) such as uridines and/or cytidines modified at the 5-position, e.g. 5-(2-amino)propyl uridine, 5-bromo uridine, or adenosines and/or guanosines modified at the 8-position, e.g. 8-bromo guanosine, or deaza nucleotides, e.g. 7-deaza-adenosine, or O- and N-alkylated nucleotides, e.g. N6-methyl adenosine can be incorporated into the siRNA. As another example, sugar-modified ribonucleotides having a 2' OH-group replaced by a group selected from H, OR, R, halo, SH, SR, NH₂, NHR, NR₂ or CN, wherein R is C₁-C₆ alkyl, alkenyl or alkynyl and halo is F, Cl, Br or I. As yet another example, the backbone may be modified to comprise modified backbone linkages such as but not limited to phosphorothioates. The siRNA may comprise modifications at the base, sugar and/or backbone, including a variety of such modifications.

Thus, siRNA molecules can be provided as and/or derived from one or more forms including, e.g., as one or more isolated small-interfering RNA (siRNA) double stranded duplexes, as longer double-stranded RNA (dsRNA), or as siRNA or dsRNA transcribed from a transcriptional cassette in a DNA plasmid. The siRNA molecules may have overhangs (e.g., 3' or 5' overhangs as described in Elbashir et al., Genes Dev., 15:188 (2001) or Nykanen et al., Cell, 107:309 (2001)), or may lack overhangs (i.e., have blunt ends). The person of ordinary skill in the art will appreciate and understand how such starting sources may be modified in order to arrive at the R- and L-forms described herein.

siRNA are targeted to genes in vivo or in vitro if all or part of the nucleotide sequence of their duplex (or double stranded) is complementary to a nucleotide sequence of the targeted gene, such as cadherin-11. siRNA made be synthesized based upon known (or predicted) nucleotide sequences of nucleic acids that encode proteins or other gene products. The sequence may be complementary to a translated or untranslated sequence in the target. The degree of complementarity between the siRNA and the target may be 100% or less than 100%, provided that sufficient identity exists to a target to mediate target-specific silencing. The art is familiar with efficacious siRNA that are less than 100% complementary to their target.

The level of silencing or interference may be measured in any number of ways, including quantitation of mRNA species and/or protein species. In some instances, mRNA quantitation is preferred particularly where the protein is intracellular or otherwise difficult to observe and/or assay. mRNA levels may be measured using RT-PCR or RACE, as an example. Protein levels may be measured using immunohistochemical staining. mRNA or protein levels may be reduced by 10%, 20%, 30%, 40%, 50%, 60%, 70%, 80%, 90%, 95%, 99%, or even 100%. Depending on the application, partial reduction (i.e., less than 100% may be sufficient) as compared to the level in the absence of the exogenously applied siRNA. In some embodiments, the level is reduced by 80% or more than 80% as compared to a control that has not been exposed to exogenously applied siRNA.

### Cadherin-11 Ribozymes:

A cadherin-11 ribozyme is an enzymatic RNA molecule capable of catalyzing the specific cleavage of cadherin-11 RNA. The cadherin-11 ribozyme binds to cadherin-11 RNA in a sequence specific manner (i.e., via sequence specific hybridization), and this is followed by endonucleolytic cleavage of the cadherin-11 RNA. Examples of ribozymes include engineered hairpin or hammerhead motif ribozymes. Ribozyme sequences complementary to a target such as cadherin-11 can be identified by scanning the target for ribozyme cleavage sites (e.g., GUA, GUU, and GUC), and then generating a sequence having about 15-20 ribonucleotides spanning the cleavage site.

### Cadherin-11 Antisense:

The cadherin-11 nucleic acid antagonist may be an antisense molecule (or oligonucleotide). Antisense oligonucleotides that selectively bind to a nucleic acid molecule encoding a cadherin-11 polypeptide, or a fragment thereof, to decrease cadherin-11 activity or function are embraced by the present invention. As used herein, the term "antisense oligonucleotide" or "antisense" describes an oligonucleotide that is an oligoribonucleotide, oligodeoxyribonucleotide, modified oligoribonucleotide, or modified oligodeoxyribonucleotide which hybridizes under physiological conditions to DNA comprising a particular gene or to an mRNA transcript of that gene and, thereby, inhibits the transcription of that gene and/or the translation of that mRNA. The antisense molecules are designed so as to interfere with transcription or translation of a target gene upon hybridization with the target gene or transcript. Those skilled in the art will recognize that the exact length of the antisense oligonucleotide and its degree of complementarity with its target will depend upon the specific target selected, including the sequence of the target and the particular bases which comprise that sequence. It is preferred that the antisense oligonucleotide be constructed and arranged so as to bind selectively with the target under physiological conditions, i.e., to hybridize substantially more to the target sequence than to any other sequence in the target cell under physiological conditions. Based upon SEQ ID NO: 1 or upon allelic or homologous genomic and/or cDNA sequences, one of skill in the art can easily choose and synthesize any of a number of appropriate antisense molecules for use in accordance with the present invention. In order to be sufficiently selective and potent for inhibition, such antisense oligonucleotides should comprise at least 10 and, more preferably, at least 15 consecutive bases which are complementary to the target, although in certain cases modified oligonucleotides as short as 7 bases in length have been used successfully as antisense oligonucleotides (Wagner et al., Nat. Med. 1(11):1116-1118, 1995). Most preferably, the antisense oligonucleotides comprise a complementary sequence of 20-30 bases.

Although oligonucleotides may be chosen which are antisense to any region of the gene or mRNA transcripts, in preferred embodiments the antisense oligonucleotides correspond to N-terminal or 5' upstream sites such as translation initiation, transcription initiation or promoter sites. In addition, 3'-untranslated regions may be targeted by antisense oligonucleotides. Targeting to mRNA splicing sites has also been used in the art but may be less preferred if alternative mRNA splicing occurs. In addition, the antisense is targeted, preferably, to sites in which mRNA secondary structure is not expected (see, e.g., Sainio et al., Cell Mol. Neurobiol. 14(5):439-457, 1994) and at which proteins are not expected to bind. Finally, although SEQ ID NO:1 discloses a cDNA sequence, one of ordinary skill in the art may easily derive the genomic DNA corresponding to this sequence. Thus, the present invention also provides for antisense oligonucleotides which are complementary to the genomic DNA corresponding to SEQ ID NO:1. Similarly, antisense to allelic or homologous cadherin-11 or alternatively, cadherin-11 counter-receptor cDNAs and genomic DNAs are enabled without undue experimentation.

In one set of embodiments, the antisense oligonucleotides of the invention may be composed of "natural" deoxyribonucleotides, ribonucleotides, or any combination thereof. That is, the 5' end of one native nucleotide and the 3' end of another native nucleotide may be covalently linked, as in natural systems, via a phosphodiester internucleoside linkage. These oligonucleotides may be prepared by art recognized methods which may be carried out manually or by an automated synthesizer. They also may be produced recombinantly by vectors.

In preferred embodiments, however, the antisense oligonucleotides of the invention also may include "modified" oligonucleotides. That is, the oligonucleotides may be modified in a number of ways which do not prevent them from hybridizing to their target but which enhance their stability or targeting or which otherwise enhance their therapeutic effectiveness.

The term "modified oligonucleotide" as used herein describes an oligonucleotide in which (1) at least two of its nucleotides are covalently linked via a synthetic internucleoside linkage (i.e., a linkage other than a phosphodiester linkage between the 5' end of one nucleotide and the 3' end of another nucleotide) and/or (2) a chemical group not normally associated with nucleic acids has been covalently attached to the oligonucleotide. Preferred synthetic internucleoside linkages are phosphorothioates, alkylphosphonates, phosphorodithioates, phosphate esters, alkylphosphonothioates, phosphoramidates, carbamates, carbonates, phosphate triesters, acetamidates, carboxymethyl esters and peptides.

The term "modified oligonucleotide" also encompasses oligonucleotides with a covalently modified base and/or sugar. For example, modified oligonucleotides include oligonucleotides having backbone sugars which are covalently attached to low molecular weight organic groups other than a hydroxyl group at the 3' position and other than a phosphate group at the 5' position. Thus modified oligonucleotides may include a 2'-O-alkylated ribose group. In addition, modified oligonucleotides may include sugars such as arabinose instead of ribose.

### Delivery of Cadherin-11 Antagonists:

Nucleic acid antagonists can be delivered to a subject alone or in association with a vector. In its broadest sense, a "vector" is any vehicle capable of facilitating: (1) delivery of a nucleic acid molecule to a target cell and/or (2) uptake of a nucleic acid molecule by a target cell. Preferably, the vectors transport the cadherin-11 nucleic acid antagonist into the target cell with reduced degradation relative to the extent of degradation that would result in the absence of the vector. Optionally, a "targeting ligand" can be attached to the vector to selectively deliver the vector to a cell which expresses on its surface the cognate receptor for the targeting ligand. Methodologies for targeting include conjugates, such as those described in U.S. Patent 5,391,723. In some instances, the nucleic acid molecules of the invention are targeted for delivery to a fibrotic tissue such as an affected lung, liver, kidney, and the like.

In general, the vectors useful in the invention are divided into two classes: biological vectors and chemical/physical vectors. Biological vectors are useful for delivery/uptake of nucleic acids to/by a target cell. Biological vectors include, but are not limited to, plasmids, phagemids, viruses, other vehicles derived from viral or bacterial sources that have been manipulated by the insertion or incorporation of the nucleic acid sequences of the invention, and additional nucleic acid fragments (e.g., enhancers, promoters) which can be attached to the nucleic acid sequences of the invention. Viral vectors are a preferred type of biological vector and include, but are not limited to, nucleic acid sequences from the following viruses: adenovirus; adeno-associated virus; retrovirus, such as moloney murine leukemia virus; harvey murine sarcoma virus; murine mammary tumor virus; rouse sarcoma virus; SV40-type viruses; polyoma viruses; Epstein-Barr viruses; papilloma viruses; herpes virus; vaccinia virus; polio virus; and RNA virus such as a retrovirus. One can readily employ other vectors not named but known in the art.

A particularly preferred virus for certain applications is the adeno-associated virus, a double-stranded DNA virus. The adeno-associated virus is capable of infecting a wide range of cell types and species and can be engineered to be replication-deficient. It further has advantages, such as heat and lipid solvent stability, high transduction frequencies in cells of diverse lineages, including hemopoietic cells, and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

In general, other preferred viral vectors are based on non-cytopathic eukaryotic viruses in which non-essential genes have been replaced with the gene of interest. Non-cytopathic viruses include retroviruses, the life cycle of which involves reverse transcription of genomic viral RNA into DNA with subsequent proviral integration into host cellular DNA. Adenoviruses and retroviruses have been approved for human gene therapy trials. In general, the retroviruses are replication-deficient (i.e., capable of directing synthesis of the desired proteins, but incapable of manufacturing an infectious particle). Such genetically altered retroviral expression vectors have general utility for the high-efficiency transduction of genes in vivo. Standard protocols for producing replication-deficient retroviruses (including the steps of incorporation of exogenous genetic material into a plasmid, transfection of a packaging cell line with plasmid, production of recombinant retroviruses by the packaging cell line, collection of viral particles from tissue culture media, and infection of the target cells with viral particles) are provided in Kriegler, M., "Gene Transfer and Expression, A Laboratory Manual," W.H. Freeman C.O., New York (1990) and Murry, E.J. Ed. "Methods in Molecular Biology," vol. 7, Humana Press, Inc., Cliffton, New Jersey (1991). Another preferred retroviral vector is the vector derived from the moloney murine leukemia virus, as described in Nabel, E.G., et al., Science, v. 249, p. 1285-1288 (1990).

In addition to the biological vectors, chemical/physical vectors are useful for delivery/uptake of nucleic acids or polypeptides to/by a target cell. As used herein, a "chemical/physical vector" refers to a natural or synthetic molecule, other than those derived from bacteriological or viral sources, capable of delivering the cadherin-11 antagonist to a cell.

A preferred chemical/physical vector for the invention is a colloidal dispersion system. Colloidal dispersion systems include lipid-based systems including oil-in-water emulsions, micelles, mixed micelles, and liposomes. A preferred colloidal system for the invention is a liposome. Liposomes are artificial membrane vessels which are useful as a delivery vector in vivo or in vitro. It has been shown that large unilamellar vessels (LUV), which range in size from 0.2 - 4.0 µM can encapsulate large macromolecules. RNA, DNA, and intact virions can be encapsulated within the aqueous interior and be delivered to cells in a biologically active form (Fraley, et al., Trends Biochem. Sci., v. 6, p. 77 (1981)). In order for a liposome to be an efficient gene transfer vector, one or more of the following characteristics should be present:
(1) encapsulation of the gene of interest at high efficiency with retention of biological activity;
(2) preferential and substantial binding to a target cell in comparison to non-target cells;
(3) delivery of the aqueous contents of the vesicle to the target cell cytoplasm at high efficiency; and (4) accurate and effective expression of genetic information.

Liposomes may be targeted to a particular tissue, by coupling the liposome to a specific ligand such as a monoclonal antibody, sugar, glycolipid, or protein specific for the particular tissue or cell type. Additionally, the vector may be coupled to a nuclear targeting peptide, which will direct the cadherin-11 modulating nucleic acid molecule to the nucleus of the host cell.

Liposomes are commercially available from Gibco BRL, for example, as LIPOFECTIN™ and LIPOFECTACE™, which are formed of cationic lipids such as N-[1-(2, 3 dioleyloxy)-propyl]-N, N, N-trimethylammonium chloride (DOTMA) and dimethyl dioctadecylammonium bromide (DDAB). Methods for making liposomes are well known in the art and have been described in many publications. Liposomes also have been reviewed by Gregoriadis, G. in Trends in Biotechnology, V. 3, p. 235-241 (1985).

In general, the cadherin-11 nucleic acid antagonists can be administered to the subject (any mammalian recipient) using the same modes of administration that currently are used for gene therapy in humans (e.g., adenovirus-mediated gene therapy). A patented procedure for performing ex vivo gene therapy is outlined in U.S. Patent 5,399,346 and in exhibits submitted in the file history of that patent, all of which are publicly available documents. In general, ex vivo gene therapy involves introduction in vitro of a functional copy of a gene or fragment thereof into a cell(s) of a subject and returning the genetically engineered cell(s) to the subject. The functional copy of the gene or fragment thereof is under operable control of regulatory elements which permit expression of the gene in the genetically engineered cell(s). Numerous transfection and transduction techniques as well as appropriate expression vectors are well known to those of ordinary skill in the art, some of which are described in PCT application WO95/00654.

As an illustrative example, a vector containing a nucleic acid molecule is delivered to a site of fibrosis in a subject who is a candidate for such gene therapy. Then, the vector genetically modifies one or more cell types in the fibrotic environment with DNA encoding, for example, cadherin-11. Such genetically modified cells are expected to interfere with cadherin-11 binding to another cadherin-11.

In an alternative embodiment, primary human cells can be obtained from a subject who is a candidate for such gene therapy. Then, such cells can be genetically engineered ex vivo with DNA encoding, for example, a full length cadherin-11. Such recombinant cells are expected to inhibit cadherin-11 mediated adhesion in vivo. In yet another example, another cell type which does not express cadherin-11 can be genetically manipulated in vitro to express a cadherin-11 antagonist and then introduced into the site of fibrosis.

Exemplary compositions that can be used to facilitate in vitro uptake of nucleic acids by a target cell include calcium phosphate and other chemical mediators of intracellular transport, microinjection compositions, electroporation and homologous recombination compositions (e.g., for integrating a nucleic acid into a preselected location within the target cell chromosome).

### Pharmaceutical Compositions, Formulation, Effective Amounts:

The disclosure further provides a pharmaceutical composition (i.e., a pharmaceutical preparation) comprising a cadherin-11 antagonist. The composition includes a pharmaceutically acceptable carrier and a cadherin-11 antagonist.

The pharmaceutical preparations, as described above, are administered in effective amounts. For therapeutic applications, it is generally that amount sufficient to achieve a medically desirable result. In general, a therapeutically effective amount is that amount necessary to delay the onset of, inhibit the progression of, or halt altogether the particular condition being treated. As an example, the effective amount may be that amount which serves to reduce, alleviate, or delay the onset of the symptoms (e.g., pain, inflammation, etc.) of the disorder being treated or prevented. The effective amount will depend upon the mode of administration, the particular condition being treated and the desired outcome. It will also depend upon the stage of the condition, the severity of the condition, the age and physical condition of the subject being treated, the nature of concurrent therapy, if any, the duration of the treatment, the specific route of administration and like factors within the knowledge and expertise of the medical practitioner. For prophylactic applications, it is that amount sufficient to delay the onset of, inhibit the progression of, or halt altogether the particular condition being prevented, and may be measured by the amount required to prevent the onset of symptoms.

Generally, doses of active compounds of the present invention would be from about 0.01 mg/kg per day to 1000 mg/kg per day, preferably from about 0.1 mg/kg to 200 mg/kg and most preferably from about 0.2 mg/kg to about 20 mg/kg, in one or more dose administrations daily, for one or more days. It is expected that doses ranging from 1-500 mg/kg, and preferably doses ranging from 1-100 mg/kg, and even more preferably doses ranging from 1-50 mg/kg, will be suitable. The preferred amount can be determined by one of ordinary skill in the art in accordance with standard practice for determining optimum dosage levels of the agent. It is generally preferred that a maximum dose of a cadherin-11 antagonist that is the highest safe dose according to sound medical judgment be used.

The cadherin-11 antagonists can be administered to a subject in need of such treatment in combination with concurrent therapy for treating fibrosis. The concurrent therapy may be invasive or non-invasive (e.g., drug therapy). Examples of drug therapies for fibrosis include but are not limited to methiazole, piperlongumine, antimycin a, thiostrepton, benzbromarone, luteolin, tolfenamic acid, ciclopirox ethanolamine, (r)-(-)-apomorphine calciferol, GBR 12909, harmol, hycanthone, flufenamic acid, halofantrine, and zardaverine, as described in U.S. Patent Publication No. 20100093613. Immunosuppressants have also been used in the treatment of fibrosis. Examples include rapamycin, methotrexate, azathioprine, cyclosporin, FK-506, CDK inhibitors, and steroids and corticosteroids such as cortisone, dexamethasone, hydrocortisone, methylprednisolone, prednisolone, prednisone, and triamcinoline. Other agents that may be used include anti-inflammatory agents such as the NSAIDs. These drug therapies are well-known to those of ordinary skill in the art and are administered by modes known to those of such skill. The drug therapies are administered in amounts which are effective to achieve the physiological goals, in combination with cadherin-11 antagonists. Thus, it is contemplated that in some instances the drug therapies may be administered in amounts which are not capable of preventing or reducing the physiological consequences of fibrosis when the drug therapies are administered alone but which are capable of reducing the consequences when administered in combination with the cadherin-11 antagonists. The cadherin-11 antagonist may be formulated with such secondary therapeutic agents or they may be formulated separately. They may be administered at the same time or at separate times. For example, the cadherin-11 antagonist may be administered before, and/or with, and/or after the secondary therapeutic agent. Alternatively, the secondary therapeutic agent may be administered before, and/or with, and/or after the cadherin-11 antagonist.

The cadherin-11 antagonists may be administered alone or in combination with the above-described drug therapies as part of a pharmaceutical composition. Such a pharmaceutical composition may include the cadherin-11 antagonist in combination with any standard physiologically and/or pharmaceutically acceptable carriers which are known in the art. The compositions should be sterile and contain a therapeutically effective amount of the cadherin-11 modulating agent in a unit of weight or volume suitable for administration to a patient.

The term "pharmaceutically-acceptable carrier" as used herein means one or more compatible solid or liquid filler, diluents or encapsulating substances which are suitable for administration into a human or other animal. The term "pharmaceutically acceptable" means a non-toxic material that does not interfere with the effectiveness of the biological activity of the active ingredients. Pharmaceutically acceptable further means a non-toxic material that is compatible with a biological system such as a cell, cell culture, tissue, or organism. The term "carrier" denotes an organic or inorganic ingredient, natural or synthetic, with which the active ingredient is combined to facilitate the application. The characteristics of the carrier will depend on the route of administration. The components of the pharmaceutical compositions also are capable of being commingled with the agents of the present disclosure, and with each other, in a manner such that there is no interaction which would substantially impair the desired pharmaceutical efficacy. The pharmaceutically acceptable carrier must be sterile for in vivo administration. Physiologically and pharmaceutically acceptable carriers include diluents, fillers, salts, buffers, stabilizers, solubilizers, and other materials which are well known in the art.

Compositions suitable for parenteral administration conveniently comprise a sterile aqueous preparation of the cadherin-11 modulating agents, which is preferably isotonic with the blood of the recipient. This aqueous preparation may be formulated according to known methods using suitable dispersing or wetting agents and suspending agents. The sterile injectable preparation also may be a sterile injectable solution or suspension in a non-toxic parenterally-acceptable diluent or solvent, for example, as a solution in 1,3-butane diol. Among the acceptable vehicles and solvents that may be employed are water, Ringer's solution, and isotonic sodium chloride solution. In addition, sterile, fixed oils are conventionally employed as a solvent or suspending medium. For this purpose, any bland fixed oil may be employed including synthetic mono- or di-glycerides. In addition, fatty acids such as oleic acid may be used in the preparation of injectables. Carrier formulations suitable for oral, subcutaneous, intravenous, intramuscular, etc. administrations can be found in Remington's Pharmaceutical Sciences, Mack Publishing Co., Easton, PA.

A variety of administration routes are available. The particular mode selected will depend, of course, upon the particular type of fibrosis being treated, the severity of the condition being treated, and the dosage required for therapeutic efficacy. The methods of the disclosure, generally speaking, may be practiced using any mode of administration that is medically acceptable, meaning any mode that produces effective levels of the active compounds without causing clinically unacceptable adverse effects. Such modes of administration include topical, oral, rectal, nasal, intranasal, inhalation, or parenteral routes. The term "parenteral" includes subcutaneous, intravenous, intramuscular, intraperitoneal, or infusion.

The pharmaceutical compositions may conveniently be presented in unit dosage form and may be prepared by any of the methods well-known in the art of pharmacy. All methods include the step of bringing the cadherin-11 antagonists into association with a carrier which constitutes one or more accessory ingredients. In general, the compositions are prepared by uniformly and intimately bringing the cadherin-11 antagonists into association with a liquid carrier, a finely divided solid carrier, or both, and then, if necessary, shaping the product. Compositions suitable for oral administration may be presented as discrete units, such as capsules, tablets, lozenges, each containing a predetermined amount of the cadherin-11 antagonist. Other compositions include suspensions in aqueous liquids or non-aqueous liquids such as a syrup, elixir or an emulsion.

In one particular embodiment, the preferred vehicle for delivery of the cadherin-11 antagonists is a biocompatible microparticle or implant that is suitable for implantation into a subject. Exemplary bioerodible implants that are useful in accordance with this method are described in PCT International application no. PCT/US/03307 (Publication No. WO 95/24929, entitled "Polymeric Gene Delivery System", claiming priority to U.S. patent application serial no. 213,668, filed March 15, 1994). PCT/US/0307 describes a biocompatible, preferably biodegradable polymeric matrix for containing an exogenous gene under the control of an appropriate promoter. The polymeric matrix is used to achieve sustained release of the exogenous gene in the subject. In accordance with the instant invention, the cadherin-11 modulating agents described herein are encapsulated or dispersed within the biocompatible, preferably biodegradable polymeric matrix disclosed in PCT/US/03307. The polymeric matrix preferably is in the form of a microparticle such as a microsphere (wherein, for example, the cadherin-11 inhibitory agent is dispersed throughout a solid polymeric matrix) or a microcapsule (wherein, for example, the cadherin-11 inhibitory agent is stored in the core of a polymeric shell). Other forms of the polymeric matrix for containing the cadherin-11 modulating agent include films, coatings, gels, implants, and stents. The size and composition of the polymeric matrix device is selected to result in favorable release kinetics in the tissue into which the matrix device is implanted. The size of the polymeric matrix devise is further selected according to the method of delivery which is to be used, including for example administration of a suspension by aerosol into the nasal and/or pulmonary areas. The polymeric matrix composition can be selected to have both favorable degradation rates and also to be formed of a material which is bioadhesive. The matrix composition also can be selected not to degrade, but rather, to release by diffusion over an extended period of time.

Both non-biodegradable and biodegradable polymeric matrices can be used to deliver the cadherin-11 antagonists to the subject. Biodegradable matrices are preferred. Such polymers may be natural or synthetic polymers. Synthetic polymers are preferred. The polymer is selected based on the period of time over which release is desired, generally in the order of a few hours to a year or longer. Typically, release over a period ranging from between a few hours and three to twelve months is most desirable. The polymer optionally is in the form of a hydrogel that can absorb up to about 90% of its weight in water and further, optionally is crosslinked with multivalent ions or other polymers.

In general, the cadherin-11 antagonists are delivered using the bioerodible implant by way of diffusion, or more preferably, by degradation of the polymeric matrix. Exemplary synthetic polymers which can be used to form the biodegradable delivery system include: polyamides, polycarbonates, polyalkylenes, polyalkylene glycols, polyalkylene oxides, polyalkylene terepthalates, polyvinyl alcohols, polyvinyl ethers, polyvinyl esters, poly-vinyl halides, polyvinylpyrrolidone, polyglycolides, polysiloxanes, polyurethanes and co-polymers thereof, alkyl cellulose, hydroxyalkyl celluloses, cellulose ethers, cellulose esters, nitro celluloses, polymers of acrylic and methacrylic esters, methyl cellulose, ethyl cellulose, hydroxypropyl cellulose, hydroxy-propyl methyl cellulose, hydroxybutyl methyl cellulose, cellulose acetate, cellulose propionate, cellulose acetate butyrate, cellulose acetate phthalate, carboxylethyl cellulose, cellulose triacetate, cellulose sulphate sodium salt, poly(methyl methacrylate), poly(ethyl methacrylate), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate), polyethylene, polypropylene, poly(ethylene glycol), poly(ethylene oxide), poly(ethylene terephthalate), poly(vinyl alcohols), polyvinyl acetate, poly vinyl chloride, polystyrene and polyvinylpyrrolidone.

Examples of biodegradable polymers include synthetic polymers such as polymers of lactic acid and glycolic acid, polyanhydrides, poly(ortho)esters, polyurethanes, poly(butic acid), poly(valeric acid), and poly(lactide-co-caprolactone), and natural polymers such as alginate and other polysaccharides including dextran and cellulose, collagen, chemical derivatives thereof (substitutions, additions of chemical groups, for example, alkyl, alkylene, hydroxylations, oxidations, and other modifications routinely made by those skilled in the art), albumin and other hydrophilic proteins, zein and other prolamines and hydrophobic proteins, copolymers and mixtures thereof. In general, these materials degrade either by enzymatic hydrolysis or exposure to water *in vivo,* by surface or bulk erosion.

Bioadhesive polymers of particular interest include bioerodible hydrogels (described by H.S. Sawhney, C.P. Pathak and J.A. Hubell in Macromolecules, 1993, 26, 581-587), polyhyaluronic acids, casein, gelatin, glutin, polyanhydrides, polyacrylic acid, alginate, chitosan, poly(methyl methacrylates), poly(ethyl methacrylates), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate).

Examples of non-biodegradable polymers include ethylene vinyl acetate, poly(meth)acrylic acid, polyamides, copolymers and mixtures thereof.

Other delivery systems can include time-release, delayed release or sustained release delivery systems. Such systems can avoid repeated administrations of the cadherin-11 antagonists described above, increasing convenience to the subject and the physician. Many types of release delivery systems are available and known to those of ordinary skill in the art. They include the above-described polymeric systems, as well as polymer base systems such as poly(lactide-glycolide), copolyoxalates, polycaprolactones, polyesteramides, polyorthoesters, polyhydroxybutyric acid, and polyanhydrides. Microcapsules of the foregoing polymers containing drugs are described in, for example, U.S. Patent 5,075,109. Delivery systems also include non-polymer systems that are: lipids including sterols such as cholesterol, cholesterol esters and fatty acids or neutral fats such as mono- di- and tri-glycerides; hydrogel release systems; silastic systems; peptide based systems; wax coatings; compressed tablets using conventional binders and excipients; partially fused implants; and the like. Specific examples include, but are not limited to: (a) erosional systems in which the cadherin-11 modulating agent is contained in a form within a matrix such as those described in U.S. Patent Nos. 4,452,775, 4,675,189 and 5,736,152 and (b) diffusional systems in which an active component permeates at a controlled rate from a polymer such as described in U.S. Patent Nos. 3,854,480, 5,133,974 and 5,407,686. In addition, pump-based hardware delivery systems can be used, some of which are adapted for implantation.

Use of a long-term sustained release implant may be particularly suitable for treatment of chronic conditions. Long-term release, are used herein, means that the implant is constructed and arranged to delivery therapeutic levels of the active ingredient for at least 30 days, and preferably 60 days. Long-term sustained release implants are well-known to those of ordinary skill in the art and include some of the release systems described above.

### Detecting Cadherin-11 Levels and Measuring Cadherin-11 Levels:

As discussed herein, the disclosure contemplates detecting cadherin-11 levels in tissues or cells of subjects in order to determine whether the subject has fibrosis (resulting in a diagnosis of fibrosis) or whether the subject is likely to develop fibrosis (resulting in a prognosis of fibrosis). Also as discussed above, diagnosis or prognosis may involve consideration of other factors such as contemporaneous symptoms of fibrosis, familial history, or patient history (including information about etiological factors known to contribute to or cause fibrosis).

Detection of cadherin-11 levels may involve detecting cadherin-11 genomic DNA levels, mRNA levels, miRNA levels, and/or protein levels. Methods for detecting any of the foregoing are known in the art, and include PCR, RT-PCR, immunohistochemistry, FACS analysis, ELISA, Southern analysis, Northern analysis, Western analysis, microarray analysis, etc. Diagnosis and/or prognosis of fibrosis may be indicated by the presence of cadherin-11 levels that are abnormally elevated in the tissue or cells of interest. Abnormally elevated levels are defined as levels that are higher than the level in a normal tissue (or a section of tissue) or cells that are non-fibrotic. Thus, in order to determine whether the level of cadherin-11 is abnormally elevated, it is typically compared to the level of cadherin-11 in a normal (non-fibrotic) tissue (or a non-fibrotic region of a tissue) or cells, or to a pre-determined normal level of cadherin-11. Pre-determined normal levels of cadherin-11 for a given tissue may be available based on population studies or other historical data. Accordingly, the comparison need not be made strictly to tissue or cells in the subject.

The cadherin-11 level in a subject is typically determined from a sample harvested from a subject. The nature of the sample will typically depend upon the type of fibrosis that is suspected of existing or developing or that is known to exist. For example, if the fibrosis is dermal fibrosis, the sample may a skin sample. Skin "punch" samples are routine in the art. As another example, if the fibrosis is lung fibrosis, the sample may be, for example, a lung biopsy, resected lung tissue, or it may be a bronchoalveolar lavage (BAL) sample. The disclosure provides, inter alia, the unexpected finding that, in subjects having lung fibrosis, cadherin-11 expressing cells include alveolar macrophages and alveolar epithelial cells, both of which can be obtained in a BAL sample. The harvest of such samples is known in the art.

An abnormally elevated level of cadherin-11 may be a level that is about 5%, about 10%, about 20%, about 30%, about 40%, about 50%, about 60%, about 70%, about 80%, about 90%, or about 100% greater than the level in a normal control sample. The degree to which the cadherin-11 level is elevated may indicate the extent of disease, such that lower elevated levels may be indicative of disease onset while higher elevated levels may be indicative of establishment of the disease.

It is to be understood that cadherin-11 can be detected and measured using the cadherin-11 antagonists described herein, as will be readily apparent to one of ordinary skill in the art.

The following Examples are included for purposes of illustration and are not intended to limit the scope of the invention.

### EXAMPLES

### Example 1:

To investigate whether cadherin-11 levels are upregulated during the process of lung fibrosis, the bleomycin-induced lung fibrosis model was used. Wild type mice (C57BL/6) were administered either saline (control) or bleomycin via the intratracheal route. Twelve days after the administration of bleomycin or saline, mice were humane sacrificed and lungs were processed for histology and western blot analyses to determine if cadherin-11 is increased during lung fibrosis in this mouse model. Protein lysates were separated on a 10% acrylamide gel by electrophoresis, then transfer to a membrane for western blotting using isotype control antibody, anti-cadherin-11 antibody (Invitrogen) or anti-GAPDH antibody. Wild type and cadherin-11 null fibroblast-like synoviocytes were used as positive and negative controls for the expression of cadherin-11. The bleomycin lung had increased levels of cadherin-11 but similar levels of GAPDH (FIG. 1). These data indicate that cadherin-11 is increased during the process of lung fibrosis.

To determine the cellular localization of cadherin-11 staining, tissue was processed for histological analyses and stained with rabbit polyclonal antibodies against cadherin-11 or isotype control. As seen in FIG. 2, lung sections from mice administered intratracheal bleomycin demonstrated prominent staining of cadherin-11 on fibroblast-like cells in the areas that are forming fibrotic foci (red stain). In saline administered lung sections, there is no staining of cadherin-11 within these areas.

Together these data demonstrate that cadherin-11 levels are increased early (day 12) in the fibrotic lungs in the bleomycin induced lung fibrosis model and strongly suggest that cadherin-11 may be involved in the development of fibrosis and serve as a therapeutic target.

### Example 2:

This Example demonstrates that cadherin-11 levels are increased in skin biopsies from scleroderma patients and lungs from patients with idiopathic pulmonary fibrosis. Using mice that genetically lack cadherin-11 and mouse models of skin and lung fibrosis, we also demonstrate that cadherin-11 is a critical mediator of fibrosis in the skin and lung. These data indicate that cadherin-11 is a therapeutic target for skin fibrosis and pulmonary fibrosis.

### METHODS:

Human subjects. For RNA isolation, skin biopsy specimens of clinically uninvolved skin were obtained from SSc patients and control patients without a history of autoimmune disease. All SSc patients fulfilled the American College of Rheumatology criteria for SSc. ¹6 Skin biopsies for immunohistological studies were obtained as paraffin embedded skin biopsies from 4 subjects with scleroderma and 4 subjects without scleroderma were obtained from the National Disease Research Interchange.

Surgical lung biopsy tissue samples were obtained from the Lung Tissue Research Consortium. Patients were classified as having mild IPF and severe IPF according to spirometry, pathological examination and high resolution CT scan. Bronchoalveolar lavage (BAL) fluid was obtained from discarded BAL obtained for routine clinical purposes in the evaluation of patients with IPF or fibrosing lung diseases. The studies were approved by the Committee for the Protection of Human Subjects at the University of Texas Health Science Center at Houston.

Mice. Female mice aged 6-10 weeks were used for these studies. Cadherin-11 null mice and control B6:129 F1 intercross mice were maintained at in the animal care facility at the University of Texas Health Science Center at Houston.¹⁷ C57BL/6 wild-type mice were raised at Jackson Laboratories (Bar Harbor, ME). All animal protocols were approved by the University of Texas Health Science Center at Houston Animal Care and Use Committee.

Bleomycin dermal fibrosis mouse model. Female mice ages 6-10 weeks were used for these experiments. Filter-sterilized bleomycin 0.02 u per mouse dissolved in phosphate-buffered saline (PBS)] (Teva Parenteral Medicines, Irvine, CA) or PBS was administered by daily subcutaneous injections into the shaved backs of mice using a 27-gauge needle for 28 days. At the end of the experiment, mice were humanely sacrificed and lesional skin was processed for analyses.

Bleomycin lung fibrosis model. 8-10 week old female mice were used for these experiments. Mice were anesthetized with avertin (250 mg/kg, intraperitoneally), and 3.5 U/kg bleomycin (Teva Parenteral Medicines, Irvine, CA) diluted in 50 µl sterile saline or saline alone was instilled intratracheally. On day 21, mice were humanely sacrificed and lungs harvested for analyses. Prior to harvesting the lungs, bronchoalveolar lavages were obtained with 3 lavages of 0.4 ml of PBS. Lungs were then infused with 10% buffered formalin at 25 cm of pressure and fixed overnight at 4° C.

Immunohistochemistry. Skin biopsies were obtained from 4 SSc patients and 4 normal subjects without a known history of autoimmune disease from the National Disease Research Interchange (Philadelphia, PA). Five µm sections were deparaffinized, rehydrated, and immersed in TBS-T buffer (Tris-buffered saline and 0.1% Tween 20), and treated with target retrieval solution (DAKO, Carpinteria, CA) at 95°C for 10 minutes. Rabbit polyclonal primary antibodies against cadherin-11 (Invitrogen Inc) or isotype matched control antibody (Abcam Inc., Cambridge, MA) were used. Bound antibodies were detected using secondary antibodies from the Dako Cytomation Envision System-HRP (3,3-diaminobenzidine tetrahydrochloride). Sections were counterstained with hematoxylin.

RNA Isolation and Quantitative Real Time PCR. Tissue biopsies frozen in RNALater (Qiagen, Valencia, CA) were used for RNA isolation. RNA was isolated using the RNeasy Fibrous Tissue Mini Kit using the manufacturer's instructions (Qiagen, Valencia, CA). RNA concentration and purity was determined using the Nanodrop method. One microgram of total RNA was used to synthesize cDNA using Quantitect reverse transcription kits using the manufacturer's instructions (Qiagen, Valencia, CA). Quantitative real-time PCR (qRT-PCR) was performed using validated TaqMan Gene Expression Assays for specific genes of interest and normalized to cyclophilin (Applied Biosystems Inc) on an Applied Biosystems 7900HT Fast Real-Time PCR System (Applied Biosystems Inc). Cyclophilin was used as an endogenous control to normalize transcript levels of total RNA of each sample. The data were analyzed with SDS 2.3 software using the comparative CT method (2-ddCT method). Fold change was calculated as 2ddCT.

Cadherin-11 siRNA Knock-Down. A549 cells (ATCC) were seeded in 96 well plates and grown overnight in DMEM containing 10% FBS and 1% antibiotics. For siRNA knockdown of Cdh11, cells were washed with antibiotic free media and subsequently incubated with Optimem (Invitrogen) with Lipofectamine RNAiMax (Invitrogen) and either non-targeting siRNA or human Cdh11 siRNA (antisense sequence: 5'-UUUGAAUGGAGUCAUAAGGUU) (SEQ ID NO:4) (Dharmacon RNAi Technologies, Thermo) for 48 hours. Cells were then trypsinized and reseeded in antibiotic free medium. Six hours later, cells were serum-starved overnight in DMEM containing 0.1% BSA. Cells were then stimulated with or without TGF-β at 10 ng/ml in DMEM + 0.1% BSA for 24 hours. RNA was isolated using Cell to Ct reagents (Ambion) according to manufacturer's instructions. Transcripts for CDH11, COL1A1, N cadherin (Cdh2), and E cadherin (Cdh1) were obtained with corresponding Taqman probes (Applied Biosystems) and presented as mean fold change versus control. Imaging of A549 cells was performed with a BX60 inverted phase contrast microscope (Olympus) for determination of cellular morphology.

### RESULTS:

Cadherin-11 upregulation in scleroderma skin. To determine if cadherin-11 was expressed at higher levels in skin biopsies from scleroderma patients compared to healthy control subjects, RNA was isolated from 3 mm skin biopsies from the upper arm of 6 scleroderma patients and 9 healthy control subjects. Expression levels of cadherin-11 and 2 fibrosis related genes (Colla1 and CTGF) were determined using quantitative real time PCR. As expected, scleroderma biopsies had higher levels of Col1a1 and CTGF compared to healthy control biopsies. (See FIG. 3.) Consistent with our hypothesis, the expression levels of cadherin-11 was also increased in scleroderma skin biopsies relative to healthy control subjects. These data demonstrate that cadherin-11 expression is increased in skin from scleroderma patients.

These data were extended and confirmed by staining the skin biopsies using an antibody specific for cadherin-11 in immunohistological studies. (See FIG. 4.) As seen in FIG. 4, staining of skin biopsies from healthy controls did not show any cadherin-11 staining in the fibroblasts in the dermis of normal skin (FIG. 4A low power magnification of normal skin, FIG.4B higher power magnification of normal skin). In contrast, cadherin-11 expression was noted (reddish-brown color) in fibroblasts located in the dermis of skin obtained from scleroderma patients (FIG. 4C low power magnification of scleroderma skin, FIG. 4D, E higher power magnification of scleroderma skin). Together these data demonstrate that cadherin-11 expression is increased in scleroderma skin relative to healthy control skin and the expression largely localizes to the dermal fibroblast of scleroderma biopsies.

Cadherin-11 deficient mice develop less dermal fibrosis. Given the increased expression of cadherin-11 in scleroderma skin, we next wanted to determine if cadherin-11 played a critical role in the development of dermal fibrosis. The bleomycin induced dermal fibrosis model is a widely accepted model of skin fibrosis and has been used to further our understanding of the pathogenesis of skin fibrosis and scleroderma. Cadherin-11 deficient mice were obtained with permission from Brigham and Women's Hospital (laboratory of Michael Brenner). These mice were injected subcutaneously with bleomycin or PBS for 28 days as described in the Methods section above. On day 28, the mice were humanely sacrificed and the skin was processed for histological studies and quantitative studies to determine the extent of dermal fibrosis and extracellular matrix deposition. As seen in FIG. 5, wild type mice (Wt) injected with bleomycin for 28 days (FIG. 5A) had an increase in the thickness of the dermal layer (arrow) compared to Wt mice injected with PBS FIG. 5C). Interestingly, cadherin-11 (cad-11 KO) mice injected with bleomycin (FIG. 5D) had an attenuated increased I dermal thickness relative to PBS injected wild type mice injected with bleomycin. These data were quantitated in FIG. 6A by measuring the dermal thickness in 5 random areas per skin biopsy. Similar to the histological pictures, these data demonstrate that cadherin-11 deficient mice develop less dermal fibrosis compared to the Wt mice when injected with bleomycin daily for 28 days. Lastly, to further quantitate the difference, the amount of collagen, an important extracellular matrix component of dermal fibrosis, was quantitated using the Sircol Assay. As seen in FIG. 6B, bleomycin injection increased the amount of collagen in wild type mice and the cadherin-11 deficient mice had a markedly attenuated increase in dermal collagen content upon injection of bleomycin into the skin. These data convincingly demonstrate that cadherin-11 is a critical molecule expressed in the skin for the development of dermal fibrosis.

Together the data in FIG. 3-6 demonstrate that cadherin-11 expression is increased in the skin of scleroderma patients and that cadherin-11 is a critical mediator of dermal fibrosis in a mouse model of scleroderma. These data strongly argue that cadherin-11 is a therapeutic target in dermal fibrosis and scleroderma.

Pulmonary Fibrosis. Expression and localization of cadherin-11 in patients with interstitial lung disease. To determine if cadherin-11 expression is increased in lungs of patients with pulmonary fibrosis similar to that in the skin of patients with scleroderma, cadherin-11 mRNA levels was assessed total RNA isolated from lungs of patients with idiopathic pulmonary fibrosis (IPF) with severe airway restriction versus IPF patients with normal lung function (mild IPF lungs were used as "control"). As seen in FIG. 7, lung tissue from severe IPF patients had increased levels of type I collagen (Col1a1), a major component of the fibrotic extracellular matrix. Consistent with our hypothesis, lung tissue from severe IPF patients had increased levels of cadherin-11.

Immunolocalization was performed to determine cell types expressing cadherin-11 in lungs of patients with IPF using rabbit polyclonal anticadherin-11 antibodies. Mild IPF patients (FIG. 6A) had expression of cadherin-11 only on alveolar macrophages. The cell type expressing cadherin-11 identified in severe IPF patients was the alveolar macrophage (FIG. 8C) but also very prominent expression on the hyperplastic alveolar epithelial cells (AECs) adjacent to fibrotic foci (FIG. 8B). Together the data from FIGs. 7 and 8 indicate an association of cadherin-11 expression with disease severity and it is expressed on both hyperplastic AECs and alveolar macrophages in the lungs of affected patients.

Expression of cadherin-11 in bleomycin-induced mouse model of pulmonary fibrosis. The intratracheal bleomycin model is a commonly utilized animal model to study mechanisms of pulmonary fibrosis and idiopathic pulmonary fibrosis. To examine the role of cadherin-11 in this model, initial characterization was performed using immunolocalization for cadherin-11 in wild type mice given intratracheal saline or bleomycin. Similar to humans subjects with IPF, immunohistochemistry on lungs from mice given bleomycin displays prominent cadherin-11 expression in the hyperplastic AECs and alveolar macrophages (FIG. 9). These results demonstrate similarities between this mouse model and humans and support analysis of these cell types in addressing the mechanism of cadherin-11-dependent fibrosis.

Contribution of cadherin-11 to pulmonary fibrosis. Given the increased expression of cadherin-11 in severe IPF lungs and lungs in the bleomycin lung fibrosis model, we next wanted to determine if cadherin-11 is involved with the development pulmonary fibrosis. To examine the contribution of cadherin-11 to pulmonary fibrosis, intratracheal bleomycin was administered to mice lacking cadherin-11 (Cdh11^{-/-}) and compared to wild type mice administered bleomycin. All endpoints were assessed at 21 days after bleomycin or saline installation. Results of H&E staining display no detectable difference in pulmonary histology between wild type (WT) and Cdh11 ^{-/-} mice given saline (FIGs. 10A and B). Examination of lung sections from wild type (WT) mice given bleomycin displays standard histopathologic features consistent with pulmonary fibrosis including inflammation, fibrotic foci and disruption of normal alveolar architecture (FIG. 10C). These histologic endpoints are substantially reduced in Cdh11 ^{-/-} mice (FIG. 10D).

Masson's trichrome stain of histological sections (matrix stains blue, cells red) demonstrate increased collagen deposition in wild type mice administered bleomycin which is decreased in Cdh11 ^{-/-} mice administered bleomycin (FIGs. 11A and B). IHC analyses for alpha-smooth muscle actin, a marker for myofibroblasts, was also performed. Myofibroblasts are a key cellular mediators in the pathogenesis of fibrosis that produce inflammatory cytokines and extracellular matrix. Wild type mice administered bleomycin had increased numbers of alpha-smooth muscle actin staining cells (FIG. 11C) while the number of alpha-smooth muscle actin staining cells of Cdh11 ^{-/-} mice (FIG. 11D) was markedly decreased relative to wild type mice. The data in FIG. 11 support the hypothesis that cadherin-11 is a critical mediator of pulmonary fibrosis in this mouse model.

To further quantitate the attenuation in pulmonary fibrosis in the Cdh11 ^{-/-} mice administered intratracheal bleomycin, collagen levels were determined using the Sircol assay in the bronchoalveolar (BAL) fluid from wild type and Cdh11 ^{-/-} mice administered saline or bleomycin. As seen in FIG. 12A, intratracheal bleomycin significantly increased the amount of collagen in BAL fluid in wild type mice. In contrast, BAL fluid from Cdh11 ^{-/-} mice administered bleomycin had a statistically significant decrease in collagen content relative to wild type mice. Lastly, we used the Ashcroft score, a commonly used scoring method to quantitate the amount of pulmonary fibrosis on H&E stained sections of lungs in the model. As seen in FIG. 12B, lungs from wild type mice administered intratracheal bleomycin significantly had a significantly higher score compared to lungs from Cdh11 ^{-/-} mice administered bleomycin. Together the data from FIGs. 10-12 clearly demonstrate the cadherin-11 is a critical mediator of pulmonary fibrosis. Given the increased levels of cadherin-11 in human IPF lungs, these data suggest that cadherin-11 is a therapeutic target for pulmonary fibrosis.

Systemic delivery of CDH11 blocking antibody improves established pulmonary fibrosis. The data present in FIGs. 10-12 clearly demonstrate that Cdh11 ^{-/-} mice have an attenuated pulmonary fibrotic response in the bleomycin induced fibrosis model. Although there are no gross histological differences in the lungs of wild type and Cdh11 ^{-/-} mice, it is important to determine if blockade of cadherin-11 also decreases pulmonary fibrosis. Furthermore, it is of interest to determine if cadherin-11 blockade is effective in the treatment of pulmonary fibrosis and not only in preventing the development of fibrosis as suggested by the Cdh11 ^{-/-} mice studies. Pulmonary fibrosis in the bleomycin model is established at least 7 days after bleomycin installation (A. Moeller et al., Int Journal of Biochem and Cell Biol. 2008). Therefore, to determine if mice with established fibrosis can be successfully treated by targeting cadherin-11, wild type mice were administered one of two systemic cadherin-11 blocking antibodies (clone 23C6 or 13C2) beginning 10 days after bleomycin installation.

Wild type mice were administered intratracheal bleomycin. On day 10, when fibrosis is already established, the mice were administered 500 ug of either 23C6, 13C2, or isotype control antibody via the intraperitoneal route followed by 100 ug of antibodies IP every other day until day 21. Mice were sacrificed and lungs were processed for histology. Lung sections, stained with H&E (FIG. 13), demonstrated that wild type mice administered bleomycin and isotype antibodies developed pulmonary fibrosis which was markedly attenuated in the wild type mice that received bleomycin followed by IP 23C6 or 13C2 starting on day 10.

Additional analyses in these experiments confirmed the ability of 23C6 and 13C2 to treat existing pulmonary fibrosis in the bleomycin model. As seen in FIG. 14, lung sections, stained with Masson's Trichrome demonstrated that lungs from wild type mice administered bleomycin and isotype antibodies developed marked increased deposition of the extracellular matrix which was markedly attenuated in the wild type mice that received bleomycin followed by IP 23C6 or 13C2 starting on day 10. In addition, staining the alpha smooth muscle actin, the marker of the myofibroblast demonstrated that the anti-cadherin-11 monoclonal antibodies, 13C2 and 23C6, effectively reduced the number of myofibroblasts in the lungs of mice administered intratracheal bleomycin compared to isotype control antibodies. Finally, soluble collagen levels as determined by the Sircol assay on BAL fluid from mice treated with 13C2 or 23C6 anti-cadherin-11 monoclonal antibodies were significantly reduced compared to mice given isotype control antibodies. These results confirm findings in Cdh11 ^{-/-} mice that CDH11 contributes to pulmonary fibrosis and demonstrates systemic delivery of cadherin-11 blocking antibody successfully treats established pulmonary fibrosis in the bleomycin induced fibrosis model.

Epithelial-to-mesenchymal transition (EMT). EMT is involved in the pathogenesis of fibrosis, and TGFbeta may play an role in this transition process. It is known that during EMT, expression of E-cadherin is reduced and/or eliminated. FIG. 15 shows that TGFbeta increases the expression of cadherin-11 in A549 cells, a lung epithelial cell line. In addition, expression of Col1a1 is increased, expression of E-cadherin is decreased (as expected), and expression of N-cadherin is also increased. FIGs. 16 and 17 show that when cadherin-11 expression is blocked in A549 cells, for example using a cahderin-11 specific siRNA, and then the cells are stimulated with TGFbeta, the upregulation of Col1a1 expression by TGFbeta is dramatically reduced. Furthermore, using phase contrast microscopy, it is clear that the cadherin-11 knock down with siRNA prevents the development of the mesenchymal phenotype induced by TGFbeta. In the presence of TGFbeta, A549 cells lose cell-to-cell contacts, become spindle shaped, and spread out. Reduction in the level of cadherin-11 with siRNA prevents these phenotypic changes, suggesting that cadherin-11 siRNA blocks EMT and that cadherin-11 is involved in and potentially mediating EMT. FIG. 18 further shows that cadherin-11 also blocks the TGFbeta-induced upregulation of SNAIL2, a key EMT transcription factor.

### REFERENCES

1. Mayes MD, Lacey JV, Jr., Beebe-Dimmer J et al. Prevalence, incidence, survival, and disease characteristics of systemic sclerosis in a large US population. Arthritis Rheum 2003; 48(8):2246-2255.
2. Wilson L. Cost-of-illness of scleroderma: the case for rare diseases. Semin Arthritis Rheum 1997; 27(2):73-84.
3. Thannickal VJ, Toews GB, White ES, Lynch JP, III, Martinez FJ. Mechanisms of pulmonary fibrosis. Annu Rev Med 2004; 55:395-417.
4. Sime PJ, O'Reilly KM. Fibrosis of the lung and other tissues: new concepts in pathogenesis and treatment. Clin Immunol 2001; 99(3):308-319.
5. Wagner GR. Asbestosis and silicosis. Lancet 1997; 349(9061):1311-1315.
6. Vanhee D, Gosset P, Wallaert B, Voisin C, Tonnel AB. Mechanisms of fibrosis in coal workers' pneumoconiosis. Increased production of platelet-derived growth factor, insulin-like growth factor type I, and transforming growth factor beta and relationship to disease severity. Am J Respir Crit Care Med 1994; 150(4):1049-1055.
7. Abid SH, Malhotra V, Perry MC. Radiation-induced and chemotherapy-induced pulmonary injury. Curr Opin Oncol 2001; 13(4):242-248.
8. Steen VD, Owens GR, Fino GJ, Rodnan GP, Medsger TA, Jr. Pulmonary involvement in systemic sclerosis (scleroderma). Arthritis Rheum 1985; 28(7):759-767.
9. Majumdar S, Li D, Ansari T et al. Different cytokine profiles in cryptogenic fibrosing alveolitis and fibrosing alveolitis associated with systemic sclerosis: a quantitative study of open lung biopsies. Eur Respir J 1999; 14(2):251-257.
10. Lewis MJ, Lewis EH, III, Amos JA, Tsongalis GJ. Cystic fibrosis. Am J Clin Pathol 2003; 120 Suppl:S3-13.
11. Elias JA, Lee CG, Zheng T, Ma B, Homer RJ, Zhu Z. New insights into the pathogenesis of asthma. J Clin Invest 2003; 111(3):291-297.
12. Coultas DB, Zumwalt RE, Black WC, Sobonya RE. The epidemiology of interstitial lung diseases. Am J Respir Crit Care Med 1994; 150(4):967-972.
13. Raghu G, Weycker D, Edelsberg J, Bradford WZ, Oster G. Incidence and prevalence of idiopathic pulmonary fibrosis. Am J Respir Crit Care Med 2006; 174(7):810-816.
14. Panos RJ, Mortenson RL, Niccoli SA, King TE, Jr. Clinical deterioration in patients with idiopathic pulmonary fibrosis: causes and assessment. Am J Med 1990; 88(4):396-404.
15. Selman M, King TE, Pardo A. Idiopathic pulmonary fibrosis: prevailing and evolving hypotheses about its pathogenesis and implications for therapy. Ann Intern Med 2001; 134(2):136-151.
16. Preliminary criteria for the classification of systemic sclerosis (scleroderma). Subcommittee for scleroderma criteria of the American Rheumatism Association Diagnostic and Therapeutic Criteria Committee. Arthritis Rheum 1980; 23(5):581-590.
17. Lee DM, Kiener HP, Agarwal SK et al. Cadherin-11 in synovial lining formation and pathology in arthritis. Science 2007; 315(5814):1006-1010.

### EQUIVALENTS

All definitions, as defined and used herein, should be understood to control over dictionary definitions, definitions in documents cited, and/or ordinary meanings of the defined terms.

All references, patents and patent applications disclosed herein are incorporated by reference with respect to the subject matter for which each is cited, which in some cases may encompass the entirety of the document.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively, as set forth in the United States Patent Office Manual of Patent Examining Procedures, Section 2111.03.

## Claims

1. A cadherin-11 antagonist:
(a) in an amount effective to reduce fibrosis for use in a method for treating a subject having fibrosis; or
(b) in an amount effective to prevent or delay the onset of symptoms associated with fibrosis for use in a method for treating a subject at risk of developing fibrosis;
wherein the cadherin-11 antagonist is a cadherin-11 binding peptide or a cadherin-11 nucleic acid antagonist.

2. A cadherin-11 antagonist for use according to claim 1, wherein the fibrosis is:
(a) dermal fibrosis;
(b) non-dermal fibrosis;
(c) lung fibrosis
(d) liver fibrosis;
(e) idiopathic pulmonary fibrosis;
(f) severe idiopathic pulmonary fibrosis

3. A cadherin-11 antagonist for use according to claim 1 or 2(a), wherein the subject has scleroderma.

4. A cadheun-11 antagonist for use according to claim 1, wherein the cadherin-11 antagonist is a binding peptide and:
(a) is an anti-cadherin-11 antibody or an antigen-binding antibody fragment;
(b) is a cadherin-11 fusion protein; or
(c) comprises full length cadherin or a fragment thereof.

5. A cadherin-11 antagonist for use according to claim 1, wherein the cadherin-11 antagonist is a nucleic acid antagonist and is:
(a) a cadherin-11 siRNA;
(b) a cadherin-11 ribozyme;
(c) a cadherin-11 antisense molecule; or
(d) a nucleic acid encoding full length cadherin-11 or a fragment thereof.

6. A cadherin-11 antagonist for use according to any one of claims 1-5, wherein the cadherin-11 antagonist is administered by inhalation, intranasally or intraperitoneally.

7. A cadherin-11 antagonist for use according to any of claims 1-6, wherein the method further comprises administering to the subject an immunosuppressant, optionally wherein the immunosuppressant is a steroid.

## Patentansprüche

1. Cadherin-11-Antagonist:
(a) in einer Menge, die wirksam ist, um Fibrose zu reduzieren, zur Verwendung in einem Verfahren zur Behandlung eines Subjekts, das Fibrose hat; oder
(b) in einer Menge, die wirksam ist, um das Auftreten von Symptomen, die mit Fibrose assoziiert sind, zu verhindern oder zu verzögern, zur Verwendung in einem Verfahren zur Behandlung eines Subjekts, das gefährdet ist, Fibrose zu entwickeln;
wobei der Cadherin-11-Antagonist ein Cadherin-11-bindendes Peptid oder ein Cadherin-11 Nukleinsäure-Antagonist ist.

2. Cadherin-11-Antagonist zur Verwendung nach Anspruch 1, wobei Fibrose:
(a) dermale Fibrose;
(b) nicht-dermale Fibrose;
(c) Lungenfibrose;
(d) Leberfibrose;
(e) idiopathische pulmonare Fibrose;
(f) schwere idiopathische pulmonare Fibrose;
ist.

3. Cadherin-11-Antagonist zur Verwendung nach Anspruch 1 oder 2(a), wobei das Subjekt Sklerodermie hat.

4. Cadherin-11-Antagonist zur Verwendung nach Anspruch 1, wobei der Cadherin-11-Antagonist ein bindendes Peptid ist und:
(a) ein anti-Cadherin-11-Antikörper oder ein Antigen-bindendes Antikörperfragment ist;
(b) ein Cadherin-11-Fusionsprotein ist; oder
(c) ein Volllängen-Cadherin oder ein Fragment davon umfasst.

5. Cadherin-11-Antagonist zur Verwendung nach Anspruch 1, wobei der Cadherin-11-Antagonist ein Nukleinsäure-Antagonist ist und:
(a) eine Cadherin-11-siRNA;
(b) ein Cadherin-11-Ribozym;
(c) ein Cadherin-11-Antisensemolekül; oder
(d) eine Nukleinsäure ist, die für Volllängen-Cadherin-11 oder ein Fragment davon kodiert,
ist.

6. Cadherin-11-Antagonist zur Verwendung nach einem der Ansprüche 1-5, wobei der Cadherin-11-Antagonist durch Inhalation, intranasal oder intraperitoneal verabreicht wird.

7. Cadherin-11-Antagonist zur Verwendung nach einem der Ansprüche 1-6, wobei das Verfahren weiter Verabreichen eines Immunsuppressivums an das Subjekt umfasst, wobei das Immunsuppressivum gegebenenfalls ein Steroid ist.

## Revendications

1. Antagoniste de cadhérine-11 :
(a) en une quantité efficace pour réduire la fibrose pour une utilisation dans un procédé pour traiter un sujet souffrant de la fibrose ; ou
(b) en une quantité efficace pour prévenir ou retarder l'apparition de symptômes associés à la fibrose pour une utilisation dans un procédé pour traiter un sujet susceptible de développer une fibrose ;
où l'antagoniste de cadhérine-11 est un peptide de liaison à la cadhérine-11 ou un antagoniste d'acide nucléique de la cadhérine-11.

2. Antagoniste de cadhérine-11 pour une utilisation selon la revendication 1, dans lequel la fibrose est :
(a) une fibrose dermique ;
(b) une fibrose non-dermique ;
(c) une fibrose pulmonaire
(d) une fibrose hépatique ;
(e) une fibrose pulmonaire idiopathique ;
(f) une fibrose pulmonaire idiopathique sévère.

3. Antagoniste de cadhérine-11 pour une utilisation selon la revendication 1 ou 2(a), dans lequel le sujet souffre d'une sclérodermie.

4. Antagoniste de cadhérine-11 pour une utilisation selon la revendication 1, dans lequel l'antagoniste de cadhérine-11 est un peptide de liaison et :
(a) est un anticorps anti-cadhérine-11 ou un fragment d'anticorps se liant à l'antigène ;
(b) est une protéine de fusion de cadhérine-11 ; ou
(c) comprend une cadhérine pleine longueur ou un fragment de celle-ci.

5. Antagoniste de cadhérine-11 pour une utilisation selon la revendication 1, dans lequel l'antagoniste de cadhérine-11 est un antagoniste d'acide nucléique et est :
(a) de l'ARNsi de cadhérine-11 ;
(b) un ribozyme de cadhérine-11 ;
(c) une molécule antisens de cadhérine-11 ; ou
(d) un acide nucléique codant pour une cadhérine-11 pleine longueur ou un fragment de celle-ci.

6. Antagoniste de cadhérine-11 pour une utilisation selon l'une quelconque des revendications 1 à 5, dans lequel l'antagoniste de cadhérine-11 est administré par inhalation, par voie intranasale ou par voie intrapéritonéale.

7. Antagoniste de cadhérine-11 pour une utilisation selon l'une des revendications 1 à 6, dans lequel le procédé comprend en outre le fait d'administrer au sujet un immunosuppresseur, facultativement dans lequel l'immunosuppresseur est un stéroïde.


### SEQUENCE LISTING

<110> The Brigham and Women's Hospital, Inc. Board of Regents, The University of Texas System Agarwal, Sandeep K. Schneider, Daniel J. Brenner, Michael B.
<120> DETECTION AND TREATMENT OF FIBROSIS
<130> B0801.70366WO00
<140> Not Yet Assigned
   <141> 2011-05-04
<150> US 61/331,355
   <151> 2010-05-04
<150> US 61/331,357
   <151> 2010-05-04
<160> 4
<170> PatentIn version 3.5
<210> 1
   <211> 3654
   <212> DNA
   <213> H. sapiens
<400> 1
   agatgccgcg ggggccgctc gcagccgccg ctgacttgtg aatgggaccg ggactggggc      60
   cgggactgac accgcagcgc ttgccctgcg ccagggactg gcggctcgga ggttgcgtcc     120
   accctcaagg gccccagaaa tcactgtgtt ttcagctcag cggccctgtg acattccttc     180
   gtgttgtcat ttgttgagtg accaatcaga tgggtggagt gtgttacaga aattggcagc     240
   aagtatccaa tgggtgaaga agaagctaac tggggacgtg ggcagccctg acgtgatgag     300
   ctcaaccagc agagacattc catcccaaga gaggtctgcg tgacgcgtcc gggaggccac     360
   cctcagcaag accaccgtac agttggtgga aggggtgaca gctgcattct cctgtgccta     420
   ccacgtaacc aaaaatgaag gagaactact gtttacaagc cgccctggtg tgcctgggca     480
   tgctgtgcca cagccatgcc tttgccccag agcggcgggg gcacctgcgg ccctccttcc     540
   atgggcacca tgagaagggc aaggaggggc aggtgctaca gcgctccaag cgtggctggg     600
   tctggaacca gttcttcgtg atagaggagt acaccgggcc tgaccccgtg cttgtgggca     660
   ggcttcattc agatattgac tctggtgatg ggaacattaa atacattctc tcaggggaag     720
   gagctggaac catttttgtg attgatgaca aatcagggaa cattcatgcc accaagacgt     780
   tggatcgaga agagagagcc cagtacacgt tgatggctca ggcggtggac agggacacca     840
   atcggccact ggagccaccg tcggaattca ttgtcaaggt ccaggacatt aatgacaacc     900
   ctccggagtt cctgcacgag acctatcatg ccaacgtgcc tgagaggtcc aatgtgggaa     960
   cgtcagtaat ccaggtgaca gcttcagatg cagatgaccc cacttatgga aatagcgcca    1020
   agttagtgta cagtatcctc gaaggacaac cctatttttc ggtggaagca cagacaggta 1080
   tcatcagaac agccctaccc aacatggaca gggaggccaa ggaggagtac cacgtggtga 1140
   tccaggccaa ggacatgggt ggacatatgg gcggactctc agggacaacc aaagtgacga 1200
   tcacactgac cgatgtcaat gacaacccac caaagtttcc gcagagcgta taccagatgt 1260
   ctgtgtcaga agcagccgtc cctggggagg aagtaggaag agtgaaagct aaagatccag 1320
   acattggaga aaatggctta gtcacataca atattgttga tggagatggt atggaatcgt 1380
   ttgaaatcac aacggactat gaaacacagg agggggtgat aaagctgaaa aagcctgtag 1440
   attttgaaac caaaagagcc tatagcttga aggtagaggc agccaacgtg cacatcgacc 1500
   cgaagtttat cagcaatggc cctttcaagg acactgtgac cgtcaagatc tcagtagaag 1560
   atgctgatga gccccctatg ttcttggccc caagttacat ccacgaagtc caagaaaatg 1620
   cagctgctgg caccgtggtt gggagagtgc atgccaaaga ccctgatgct gccaacagcc 1680
   cgataaggta ttccatcgat cgtcacactg acctcgacag atttttcact attaatccag 1740
   aggatggttt tattaaaact acaaaacctc tggatagaga ggaaacagcc tggctcaaca 1800
   tcactgtctt tgcagcagaa atccacaatc ggcatcagga agccaaagtc ccagtggcca 1860
   ttagggtcct tgatgtcaac gataatgctc ccaagtttgc tgccccttat gaaggtttca 1920
   tctgtgagag tgatcagacc aagccacttt ccaaccagcc aattgttaca attagtgcag 1980
   atgacaagga tgacacggcc aatggaccaa gatttatctt cagcctaccc cctgaaatca 2040
   ttcacaatcc aaatttcaca gtcagagaca accgagataa cacagcaggc gtgtacgccc 2100
   ggcgtggagg gttcagtcgg cagaagcagg acttgtacct tctgcccata gtgatcagcg 2160
   atggcggcat cccgcccatg agtagcacca acaccctcac catcaaagtc tgcgggtgcg 2220
   acgtgaacgg ggcactgctc tcctgcaacg cagaggccta cattctgaac gccggcctga 2280
   gcacaggcgc cctgatcgcc atcctcgcct gcatcgtcat tctcctggtc attgtagtat 2340
   tgtttgtgac cctgagaagg caaaagaaag aaccactcat tgtctttgag gaagaagatg 2400
   tccgtgagaa catcattact tatgatgatg aagggggtgg ggaagaagac acagaagcct 2460
   ttgatattgc caccctccag aatcctgatg gtatcaatgg atttatcccc cgcaaagaca 2520
   tcaaacctga gtatcagtac atgcctagac ctgggctccg gccagcgccc aacagcgtgg 2580
   atgtcgatga cttcatcaac acgagaatac aggaggcaga caatgacccc acggctcctc 2640
   cttatgactc cattcaaatc tacggttatg aaggcagggg ctcagtggcc gggtccctga 2700
   gctccctaga gtcggccacc acagattcag acttggacta tgattatcta cagaactggg 2760
   gacctcgttt taagaaacta gcagatttgt atggttccaa agacactttt gatgacgatt 2820
   cttaacaata acgatacaaa tttggcctta agaactgtgt ctggcgttct caagaatcta 2880
   gaagatgtgt aaacaggtat ttttttaaat caaggaaagg ctcatttaaa acaggcaaag 2940
   ttttacagag aggatacatt taataaaact gcgaggacat caaagtggta aatactgtga 3000
   aatacctttt ctcacaaaaa ggcaaatatt gaagttgttt atcaacttcg ctagaaaaaa 3060
   aaaacacttg gcatacaaaa tatttaagtg aaggagaagt ctaacgctga actgacaatg 3120
   aagggaaatt gtttatgtgt tatgaacatc caagtctttc ttctttttta agttgtcaaa 3180
   gaagcttcca caaaattaga aaggacaaca gttctgagct gtaatttcgc cttaaactct 3240
   ggacactcta tatgtagtgc atttttaaac ttgaaatata taatattcag ccagcttaaa 3300
   cccatacaat gtatgtacaa tacaatgtac aattatgtct cttgagcatc aatcttgtta 3360
   ctgctgattc ttgtaaatct ttttgcttct actttcatct taaactaata cgtgccagat 3420
   ataactgtct tgtttcagtg agagacgccc tatttctatg tcatttttaa tgtatctatt 3480
   tgtacaattt taaagttctt attttagtat acgtataaat atcagtattc tgacatgtaa 3540
   gaaaatgtta cggcatcaca cttatatttt atgaacattg tactgttgct ttaatatgag 3600
   cttcaatata agaagcaatc tttgaaataa aaaaagattt ttttttaaaa aaaa 3654
<210> 2
   <211> 796
   <212> PRT
   <213> H. sapiens
<400> 2
   Met Lys Glu Asn Tyr Cys Leu Gln Ala Ala Leu Val Cys Leu Gly Met
   1               5                   10                  15
   Leu Cys His Ser His Ala Phe Ala Pro Glu Arg Arg Gly His Leu Arg
               20                  25                  30
   Pro Ser Phe His Gly His His Glu Lys Gly Lys Glu Gly Gln Val Leu
           35                  40                  45
   Gln Arg Ser Lys Arg Gly Trp Val Trp Asn Gln Phe Phe Val Ile Glu
       50                  55                  60
   Glu Tyr Thr Gly Pro Asp Pro Val Leu Val Gly Arg Leu His Ser Asp
   65                  70                  75                  80
   Ile Asp Ser Gly Asp Gly Asn Ile Lys Tyr Ile Leu Ser Gly Glu Gly
                   85                  90                  95
   Ala Gly Thr Ile Phe Val Ile Asp Asp Lys Ser Gly Asn Ile His Ala
               100                 105                 110
   Thr Lys Thr Leu Asp Arg Glu Glu Arg Ala Gln Tyr Thr Leu Met Ala
           115                 120                 125
   Gln Ala Val Asp Arg Asp Thr Asn Arg Pro Leu Glu Pro Pro Ser Glu
       130                 135                 140
   Phe Ile Val Lys Val Gln Asp Ile Asn Asp Asn Pro Pro Glu Phe Leu
   145 150 155 160
   His Glu Thr Tyr His Ala Asn Val Pro Glu Arg Ser Asn Val Gly Thr
   165 170 175
   Ser Val Ile Gln Val Thr Ala Ser Asp Ala Asp Asp Pro Thr Tyr Gly
   180 185 190
   Asn Ser Ala Lys Leu Val Tyr Ser Ile Leu Glu Gly Gln Pro Tyr Phe
   195 200 205
   Ser Val Glu Ala Gln Thr Gly Ile Ile Arg Thr Ala Leu Pro Asn Met
   210 215 220
   Asp Arg Glu Ala Lys Glu Glu Tyr His Val Val Ile Gln Ala Lys Asp
   225 230 235 240
   Met Gly Gly His Met Gly Gly Leu Ser Gly Thr Thr Lys Val Thr Ile
   245 250 255
   Thr Leu Thr Asp Val Asn Asp Asn Pro Pro Lys Phe Pro Gln Ser Val
   260 265 270
   Tyr Gln Met Ser Val Ser Glu Ala Ala Val Pro Gly Glu Glu Val Gly
   275 280 285
   Arg Val Lys Ala Lys Asp Pro Asp Ile Gly Glu Asn Gly Leu Val Thr
   290 295 300
   Tyr Asn Ile Val Asp Gly Asp Gly Met Glu Ser Phe Glu Ile Thr Thr
   305 310 315 320
   Asp Tyr Glu Thr Gln Glu Gly Val Ile Lys Leu Lys Lys Pro Val Asp
   325 330 335
   Phe Glu Thr Lys Arg Ala Tyr Ser Leu Lys Val Glu Ala Ala Asn Val
   340 345 350
   His Ile Asp Pro Lys Phe Ile Ser Asn Gly Pro Phe Lys Asp Thr Val
   355 360 365
   Thr Val Lys Ile Ser Val Glu Asp Ala Asp Glu Pro Pro Met Phe Leu
   370 375 380
   Ala Pro Ser Tyr Ile His Glu Val Gln Glu Asn Ala Ala Ala Gly Thr
   385 390 395 400
   Val Val Gly Arg Val His Ala Lys Asp Pro Asp Ala Ala Asn Ser Pro
                   405                 410                 415
   Ile Arg Tyr Ser Ile Asp Arg His Thr Asp Leu Asp Arg Phe Phe Thr
               420                 425                 430
   Ile Asn Pro Glu Asp Gly Phe Ile Lys Thr Thr Lys Pro Leu Asp Arg
           435                 440                 445
   Glu Glu Thr Ala Trp Leu Asn Ile Thr Val Phe Ala Ala Glu Ile His
       450                 455                 460
   Asn Arg His Gln Glu Ala Lys Val Pro Val Ala Ile Arg Val Leu Asp
   465                 470                 475                 480
   Val Asn Asp Asn Ala Pro Lys Phe Ala Ala Pro Tyr Glu Gly Phe Ile
                   485                 490                 495
   Cys Glu Ser Asp Gln Thr Lys Pro Leu Ser Asn Gln Pro Ile Val Thr
               500                 505                 510
   Ile Ser Ala Asp Asp Lys Asp Asp Thr Ala Asn Gly Pro Arg Phe Ile
           515                 520                 525
   Phe Ser Leu Pro Pro Glu Ile Ile His Asn Pro Asn Phe Thr Val Arg
       530                 535                 540
   Asp Asn Arg Asp Asn Thr Ala Gly Val Tyr Ala Arg Arg Gly Gly Phe
   545                 550                 555                 560
   Ser Arg Gln Lys Gln Asp Leu Tyr Leu Leu Pro Ile Val Ile Ser Asp
                   565                 570                 575
   Gly Gly Ile Pro Pro Met Ser Ser Thr Asn Thr Leu Thr Ile Lys Val
               580                 585                 590
   Cys Gly Cys Asp Val Asn Gly Ala Leu Leu Ser Cys Asn Ala Glu Ala
           595                 600                 605
   Tyr Ile Leu Asn Ala Gly Leu Ser Thr Gly Ala Leu Ile Ala Ile Leu
       610                 615                 620
   Ala Cys Ile Val Ile Leu Leu Val Ile Val Val Leu Phe Val Thr Leu
   625                 630                 635                 640
   Arg Arg Gln Lys Lys Glu Pro Leu Ile Val Phe Glu Glu Glu Asp Val
                   645                 650                 655
   Arg Glu Asn Ile Ile Thr Tyr Asp Asp Glu Gly Gly Gly Glu Glu Asp
   660 665 670
   Thr Glu Ala Phe Asp Ile Ala Thr Leu Gln Asn Pro Asp Gly Ile Asn
   675 680 685
   Gly Phe Ile Pro Arg Lys Asp Ile Lys Pro Glu Tyr Gln Tyr Met Pro
   690 695 700
   Arg Pro Gly Leu Arg Pro Ala Pro Asn Ser Val Asp Val Asp Asp Phe
   705 710 715 720
   Ile Asn Thr Arg Ile Gln Glu Ala Asp Asn Asp Pro Thr Ala Pro Pro
   725 730 735
   Tyr Asp Ser Ile Gln Ile Tyr Gly Tyr Glu Gly Arg Gly Ser Val Ala
   740 745 750
   Gly Ser Leu Ser Ser Leu Glu Ser Ala Thr Thr Asp Ser Asp Leu Asp
   755 760 765
   Tyr Asp Tyr Leu Gln Asn Trp Gly Pro Arg Phe Lys Lys Leu Ala Asp
   770 775 780
   Leu Tyr Gly Ser Lys Asp Thr Phe Asp Asp Asp Ser
   785 790 795
<210> 3
   <211> 34
   <212> PRT
   <213> H. sapiens
<400> 3
   Gly Trp Val Trp Asn Gln Phe Phe Val Ile Glu Glu Tyr Thr Gly Pro
   1 5 10 15
   Asp Pro Val Leu Val Gly Arg Leu His Ser Asp Ile Asp Ser Gly Asp
   20 25 30
   Gly Asn
<210> 4
   <211> 21
   <212> RNA
   <213> artificial sequence
<220>
   <223> human Cdh11 siRNA
<400> 4
   uuugaaugga gucauaaggu u 21